# EUROPEAN PATENT SPECIFICATION

(11) **EP 3 421 588 B1**
(45) Date of publication and mention of the grant of the patent: **10.04.2024**
(21) Application number: 17756562.9
(22) Date of filing: 22.02.2017
(51) Int. Cl.: C12N 5/07, C12N 5/0793, C12N 5/071, C12N 5/00, C12N 5/077, C12N 5/09

(54) **METHOD FOR PRODUCING THREE-DIMENSIONAL CELL TISSUE**
VERFAHREN ZUR HERSTELLUNG VON DREIDIMENSIONALEM ZELLGEWEBE
PROCÉDÉ DE PRODUCTION DE TISSU CELLULAIRE TRIDIMENSIONNEL

(30) Priority: 22.02.2016 JP 2016030916
(43) Date of publication of application: 02.01.2019
(60) Divisional application: 24159075.1
(73) Proprietor: Osaka University, Suita-shi, Osaka 565-0871 (JP); Toppan Printing Co., Ltd., Taito-ku, Tokyo 110-0016 (JP)
(72) Inventor: MATSUSAKI, Michiya, Suita-shi Osaka 565-0871 (JP); IRIE, Shinji, Tokyo 110-0016 (JP); KITANO, Shiro, Tokyo 110-0016 (JP)
(74) Representative: TBK
(86) International application number: PCT/JP2017/006691
(87) International publication number: WO 2017/146124

(56) References cited:
- WO-A1-2014/174899
- JP-A- 2012 115 254
- JP-A- 2012 205 516
- AKIHIRO NISHIGUCHI ET AL: "Cell-Cell Crosslinking by Bio-Molecular Recognition of Heparin-Based Layer-by-Layer Nanofilms : Cell-Cell Crosslinking by Bio-Molecular Recognition of Heparin-Based Layer-by-Layer Nanofilms", MACROMOLECULAR BIOSCIENCE, vol. 15, no. 3, 9 January 2015 (2015-01-09), pages 312-317, XP055540432, DE ISSN: 1616-5187, DOI: 10.1002/mabi.201400415
- AKIHIRO NISHIGUCHI ET AL: "Rapid Construction of Three-Dimensional Multilayered Tissues with Endothelial Tube Networks by the Cell-Accumulation Technique", ADVANCED MATERIALS, WILEY-VCH GERMANY, DE, vol. 23, no. 31, 16 August 2011 (2011-08-16), pages 3506-3510, XP002753112, ISSN: 0935-9648, DOI: 10.1002/ADMA.201101787 [retrieved on 2011-07-04]
- MICHIYA MATSUSAKI ET AL: "3D-fibroblast tissues constructed by a cell-coat technology enhance tight-junction formation of human colon epithelial cells", BIOCHEMICAL AND BIOPHYSICAL RESEARCH COMMUNICATIONS, vol. 457, no. 3, 8 January 2015 (2015-01-08), pages 363-369, XP055596198, AMSTERDAM, NL ISSN: 0006-291X, DOI: 10.1016/j.bbrc.2014.12.118
- RUI R. COSTA ET AL: "Polyelectrolyte multilayered assemblies in biomedical technologies", CHEMICAL SOCIETY REVIEWS, vol. 43, no. 10, 1 January 2014 (2014-01-01), page 3453, XP055596203, UK ISSN: 0306-0012, DOI: 10.1039/c3cs60393h
- GUICAI LI ET AL: "Layer-by-layer construction of the heparin/fibronectin coatings on titanium surface:stability and functionality", PHYSICS PROCEDIA, vol. 18, 1 January 2011 (2011-01-01), pages 112-121, XP028258125, ISSN: 1875-3892, DOI: 10.1016/J.PHPRO.2011.06.068 [retrieved on 2011-08-07]
- NISHIGUCHI A. et al.: "Cell - cell crosslinking by bio-molecular recognition of heparin-based layer-by-layer nanofilms", Macromolecular Bioscience, vol. 15, 2015, pages 312-317, XP055540432,
- Strudwick et al.: "Combination of Low Calcium with Y-27632 Rock Inhibitor Increases the Proliferative Capacity, Expansion Potential and Lifespan of Primary Human Keratinocytes while Retaining Their Capacity to Differentiate into Stratified Epidermis in a 3D Skin Model", PLoS One, vol. 10, no. 4, 2015, pages 1-12, XP055315198,
- MATSUZAWA A. et al.: "Effectiveness of nanometer-sized extracellular matrix layer-by- layer assembled films for a cell membrane coating protecting cells from physical stress", Langmuir, vol. 29, 2013, pages 7362-7368, XP055540440,
- PANORCHAN P. et al.: "Microrheology and ROCK signaling of human endothelial cells embedded in a 3D Matrix", Biophysical Journal, vol. 91, 2006, pages 3499-3507, XP055540441,

## Description

### Field of the Invention

The present invention relates to a method for producing a three-dimensional cell tissue.

Priority is claimed on Japanese Patent Application No. 2016-030916, filed on February 22, 2016.

### Description of Related Art

In recent years, it has been suggested that, in drug assay systems requiring an environment which is similar to a living organism as well as regeneration medicine, it is advantageous to use a three-dimensional cell tissue which is three-dimensionally organized rather than a cell grown on a flat plate. For this reason, various techniques for constructing a three-dimensional tissue of a cell in vitro are developed. For example, a method for forming an aggregation on a surface substrate to which a cell cannot adhere (Patent Document 1), a method for forming an aggregation in liquid droplets (Patent Document 2), a method for gathering cells on a permeable membrane (Patent Document 3), and the like are developed. For maintaining such organization of cells, extracellular matrix (ECM) such as collagen which is produced by a living organism itself is required for bond between cells or formation of a scaffold. For this reason, it has been considered that the ECM is added from outside when artificially constructing a cell tissue (Patent Documents 4 to 7). Patent Document 5 discloses a method for bringing a cell isolated by enzyme treatment and the like into contact with collagen which is a representative ECM and a water-soluble polymer having a cell protecting action, and then culturing the resultant as a three-dimensional aggregate. According to the method, water-soluble polymeric gel is formed around cells during culture.

Patent Document 8 and Non-Patent Document 1 disclose a method for preparing cell sheets using a culture dish in which poly(N-isopropylacrylamide) (PIPAAm) which is a temperature-responsive resin is immobilized on the surface, and constructing a three-dimensional tissue by stacking the prepared cell sheets. In this method, peeling-off of the cell sheets is essential, but there is a difficulty in performing peeling while maintaining the shape of the cells.

Non-Patent Document 2 discloses a method for constructing a three-dimensional tissue of a cell by using magnetite cationic liposomes (MCL) containing nanomagnetic fine particles having electrostatic interaction with a cell membrane. However, in this method, there is a difficulty in controlling the thickness or three-dimensional arrangement of a cell layer.

Patent Document 9 discloses a method for constructing a three-dimensional tissue by repeatedly performing a step for forming a cell layer and bringing the cell layer into contact with a first substance-containing liquid and a second substance-containing liquid, respectively, and consecutively stacking the cell layer via an extracellular matrix (ECM) having a thickness of a nanometer size. In this method, since peeling of a cell sheet of a single layer, overlapping of a peeled cell sheet, or the like is not necessary, it is possible to produce a three-dimensional tissue with excellent reproducibility and efficiency. However, since one cell layer is cultured at a time, it takes long time to construct the three-dimensional tissue. In addition, since it is difficult to arrange various cells in the same plane, there is a limitation to variation of three-dimensional arrangement.

Patent Document 10 discloses a method for constructing a three-dimensional tissue by preparing a coated cell of which the whole surface is coated with an adhesive membrane and adhering the cell via the adhesive membrane. In this method, a lot of times of centrifugal separation steps are necessary for preparation of the coated cell. For this reason, there is a possibility that the cell receives physical damage depending on the kind of the cell. In addition, there is a case where the cell is lost along with removal of a supernatant during centrifugal separation, and thus cell recovery rate is lowered.

The present inventors previously found that, by mixing normal human thermal fibroblast (NHDF) cells with fibronectin (FN), heparin (Hep), or dextran sulfate (DS) and subsequently subjecting the mixture to centrifugal separation, a gel-form cell aggregate is formed (Non-Patent Document 3). In addition, a three-dimensional tissue was constructed by using the gel-form cell aggregate to the method disclosed in Patent Document 10. However, in the above-described method, there is a difficulty in obtaining a thick tissue.

AKIHIRO NISHIGUCHI et al., "Rapid Construction of Three-Dimensional Multilayered Tissues with Endothelial Tube Networks by the Cell-Accumulation Technique", ADVANCED MATERIALS, WILEY-VCH GERMANY, DE, vol. 23, no. 31, pages 3506 - 3510 describes a cell-accumulation technique using highly biocompatible nanofilms by layer-by-layer assembly for the rapid construction of thick layered tissues with a well-controlled layer number and thickness. Furthermore, 3D tissues with highly developed blood capillary networks (over 1 cm² of layered tissues) were also constructed by sandwiching endothelial cells between the layered tissues.

MICHIYA MATSUSAKI et al., "3D-fibroblast tissues constructed by a cell-coat technology enhance tight-junction formation of human colon epithelial cells", BIOCHEMICAL AND BIOPHYSICAL RESEARCH COMMUNICATIONS, AMSTERDAM, NL, (20150108), vol. 457, no. 3, pages 363 - 369 describes an enhancement in tight-junction formation by 3D-cultures of Caco-2 cells on monolayered (1L) and eight layered (8L) normal human dermal fibroblasts (NHDF).

In RUI R. COSTA et al., "Polyelectrolyte multilayered assemblies in biomedical technologies", CHEMICAL SOCIETY REVIEWS, UK, (20140101), vol. 43, no. 10, page 3453 the potential uses of LbL films in biomedical engineering based mainly on the assembly of polyelectrolytes are reviewed.

### Document of Related Art

### Patent Document

[Patent Document 1] Japanese Patent No. 4,159,103
[Patent Document 2] Japanese Patent No. 5,847,733
[Patent Document 3] Japanese Patent No. 5,669,741
[Patent Document 4] Japanese Unexamined Patent Application, First Publication No. S63-222685
[Patent Document 5] Japanese Patent No. 2,824,081
[Patent Document 6] Japanese Patent No. 5,458,259
[Patent Document 7] Japanese Patent No. 5,409,009
[Patent Document 8] PCT International Publication No. WO 2002/008387
[Patent Document 9] Japanese Patent No. 4,919,464
[Patent Document 10] Japanese Patent No. 5,850,419

### Non-Patent Document

[Non-Patent Document 1] Joseph Yang et al., Biomaterials, 26, 2005, 6415-6422
[Non-Patent Document 2] Akira Ito et al., Tissue engineering, 10 (5-6), 2004, 833-840
[Non-Patent Document 3] Akihiro Nishiguchi et al., Macromol Biosci. 2015 Mar; 15(3): 312-7

### SUMMARY OF THE INVENTION

### Technical Problem

An object of the present invention is to provide a method capable of producing a thicker three-dimensional cell tissue in a faster and convenient manner compared to the related art.

The present inventors performed intensive studies to achieve the above object and found a method capable of producing a thicker three-dimensional cell tissue in a faster and convenient manner compared to the related art that requires a lot of times of centrifugal treatment by using a mixture obtained by mixing cells with a cationic substance, a polymeric electrolyte, and an extracellular matrix component, thereby completing the present invention.

A method of producing a three-dimensional cell tissue according to a first aspect of the present invention is defined in claim 1.

In the first aspect, a step A'-1 of removing a liquid portion from the obtained mixture to obtain a cell aggregate and a step A'-2 of suspending the cell aggregate in a solution to obtain a suspension may be further included after the step A, and a step B' of precipitating the cells from the obtained suspension to form a cell precipitate on the substrate may be included instead of the step B.

In the first aspect, in the step B or the step A'-1, the cell aggregate may be a slurry viscous body.

In the first aspect, in the step A'-1, a method for removing the liquid portion may be centrifugal separation or filtration.

In the first aspect, in the step B or the step B', a method for gathering the cells may be centrifugal separation, magnetic separation, or filtration.

In the first aspect, the cell aggregate in the step B or the cell aggregate in the step B' may be in a layer shape.

In the first aspect, a mixture ratio of the polymeric electrolyte to the extracellular matrix component may be 1:2 to 2:1.

In the first aspect, the cells in the step A may be a plurality of kinds of cells.

In the first aspect, the plurality of kinds of cells may be selected from the group consisting of nerve cells, dendritic cells, immune cells, vascular endothelial cells, lymphatic endothelial cells, fibroblasts, cancer cells, cancer stem cells, epithelial cells, myocardial cells, liver cells, pancreatic islet cells, tissue stem cells, iPS cells, ES cells, and smooth muscle cells.

In the first aspect, a thickness of the obtained three-dimensional cell tissue may be 5 to 500 µm.

In the first aspect, the number of cell layers in the obtained three-dimensional cell tissue may be 1 to 100 layers.

In the first aspect, the number of cells per area of 100 µm in a thickness direction and of 50 µm in a width direction in a region including a position in which a thickness of the obtained three-dimensional cell tissue is the maximum may be 70 or less.

In the first aspect, in the step C, the cells may be cultured in the presence of a ROCK inhibitor.

In the first aspect, the number of cell layers per thickness of 10 µm of the obtained three-dimensional cell tissue may be 2.8 layers or less.

In the first aspect, the obtained three-dimensional cell tissue may include a plurality of kinds of cells.

In the first aspect, the obtained three-dimensional cell tissue may have a vasculature.

The use of a kit according to a second aspect of the present invention is defined in claim 13.

According to each of the aspects of the present invention, it is possible to produce a three-dimensional cell tissue in a faster and convenient manner compared to the related art. In addition, it is possible to produce a thicker three-dimensional cell tissue compared to the related art by using a method of each of the aspects of the present invention. Accordingly, it is possible to produce a three-dimensional cell tissue with a thickness that could have not been constructed until now.

### BRIEF DESCRIPTION OF THE DRAWINGS

FIG. 1 shows a result of HE stain of a three-dimensional cell tissue constructed by using heparin and collagen.
FIG. 2 shows a result of HE stain of a three-dimensional cell tissue constructed by heparin and collagen. In Sample A, a mixture of cells with heparin and collagen was sowed into a cell culture insert. In Sample B, a viscous body obtained by performing centrifugation on the mixture of cells with heparin and collagen was used.
FIG. 3 shows a result of HE stain of a three-dimensional cell tissue constructed by using heparin and collagen.
FIG. 4 shows a result of fluorescence microscope observation of a three-layer tissue body and a five-layer tissue body constructed by continuous stacking.
FIG. 5 shows a result of HE stain and a result of fluorescence microscope observation of a cancer tissue model.
FIG. 6 shows a result of examination of suppression of contraction of a tissue. FIG. 6(a) shows 3.5 × 10⁶ cells/10% FBS-containing DMEM, FIG. 6(b) shows 3.5 × 10⁶ cells/10% FBS-containing DMEM (5 µM Y-27632 addition), and FIG. 6(c) shows 5.0 × 10⁶ cells/10% FBS-containing DMEM (5 µM Y-27632 addition).
FIG. 7 shows a result of fluorescence microscope observation of a three-dimensional cell tissue having a vasculature. FIG. 7(a) shows an entire image of cell culture insert, and FIG. 7(b) shows an enlarged view of an area surrounded by a square in FIG. 7(a).
FIG. 8 shows a result of HE stain and immune stain of a three-dimensional cell tissue having vasculature.
FIG. 9 shows a tissue constructed without using heparin and collagen (Comparative Example 1).
FIG. 10 shows a tissue in a case of using a method disclosed in Patent Document 5 (Comparative Example 2). FIG. 10(a) shows an entire view of an obtained tissue, and FIG. 10(b) shows an enlarged view thereof. NHDF of 1 × 10⁵ cells were used.
FIG. 11 shows a tissue in a case of using a method disclosed in Patent Document 5 (Comparative Example 2). FIG. 10(a) shows an entire view of an obtained tissue, and FIG. 10(b) shows an enlarged view thereof. NHDF of 1 × 10⁶ cells were used.
FIG. 12 shows the tissue in a case of using a method disclosed in Patent Document 5 (Comparative Example 2). FIG. 11(a) shows an entire view of an obtained tissue, and FIG. 11(b) shows an enlarged view thereof. NHDF of 3.5 × 10⁶ cells were used.

### DETAILED DESCRIPTION OF THE INVENTION

A method of producing a three-dimensional cell tissue according to an embodiment of the present invention as defined in claim 1 relates to a method including:
(A) a step of mixing cells with a cationic substance and an extracellular matrix component,
(B) a step of gathering the cells from the obtained mixture and forming a cell aggregate on a substrate, and
(C) a step of culturing the cells and obtaining a three-dimensional cell tissue.

In the present invention, in the step (A), by mixing cells with a cationic substance and an extracellular matrix component and forming a cell aggregate from the cell mixture, it is possible to obtain a three-dimensional cell tissue in which there is a small amount of large voids inside. In addition, since the obtained three-dimensional cell tissue is relatively stable, culture for at least several days is possible, and the tissue is hardly collapsed even during culture medium exchange.

In the step (A), the cells are further mixed with a polymeric electrolyte. By mixing the cells with a cationic substance, a polymeric electrolyte, and an extracellular matrix component, a three-dimensional cell tissue which has a small number of voids, and is thick is obtained more efficiently.

The method according to the embodiment may include (A'-1) a step of removing a liquid portion from the obtained mixture and obtaining a cell aggregate and (A'-2) a step of suspending the cell aggregate in a solution, after the step (A), and (B') a step of precipitating the cell from the obtained suspension and forming a cell precipitate on the substrate, instead of the step (B). It is possible to obtain a desired tissue by carrying out the above-described steps (A) to (C). In addition, it is possible to obtain a more homogeneous tissue by performing the steps (A'-1) and (A'-2) after the step (A) and performing the step (B') instead of the step (B).

In the method of the present embodiment, mixing of the cells with the cationic substance, the polymeric electrolyte, and the extracellular matrix component may be performed in a suitable container such as dish, tube, flask, bottle, and plate, or may be performed on the substrate used in the step (B). In addition, suspension in the step (A'-2) may be performed in a suitable container such as dish, tube, flask, bottle, and plate, or may be performed on the substrate used in the step (B').

In the present specification, the "three-dimensional cell tissue" means a three-dimensional aggregate including at least one kind of cell. Examples of the three-dimensional cell tissue constructed by the present embodiment include tissues of a living organism such as skin, hair, bones, cartilage, teeth, cornea, blood vessels, lymphatic vessels, heart, liver, pancreas, nerves, and esophagus, and solid cancer models (for example, gastric cancer, esophageal cancer, colorectal cancer, colonic cancer, rectal cancer, pancreatic cancer, breast cancer, ovarian cancer, prostate cancer, nephrocyte cancer, hepatoma, and the like), and are not limited thereto.

In the present specification, the "cell aggregate" means an aggregation of cells. The cell aggregate also includes a cell precipitate (cell aggregate formed by precipitating cells) obtained by centrifugal separation or filtration. In one embodiment, the cell aggregate is a slurry viscous body. In the present specification, the "slurry viscous body" indicates a cell aggregates as disclosed in Akihiro Nishiguchi et al., Macromol Biosci. 2015 Mar; 15(3): 312-7 (Non-Patent Document 3).

As the cationic substance used in the present embodiment, an optional substance having a positive charge may be used as long as the substance does not have an adverse effect on the growth of cells and the formation of the cell aggregate. Examples of the cationic substance include cationic buffer solutions such as tris-hydrochloric acid buffer solution, tris-maleic acid buffer solution, bis-tris-buffer solution, and HEPES, ethanolamine, diethanolamine, triethanolamine, polyvinylamine, polyarylamine, polylysine, polyhistidine, and polyarginine, and are not limited thereto. The cationic substance used in the present embodiment is preferably a cationic buffer solution. The cationic substance used in the present embodiment is more preferably a tris-hydrochloric acid buffer solution.

A concentration of the cationic substance is not particularly limited as long as the concentration of the cationic substance does not have an adverse effect on the growth of the cells and the formation of the cell aggregate. The concentration of the cationic substance used in the present embodiment is preferably from 10 to 100 mM. For example, the concentration of the cationic substance used in the present embodiment is preferably from 20 to 90 mM, from 30 to 80 mM, from 40 to 70 mM, and from 45 to 60 mM. The concentration of the cationic substance used in the present embodiment is more preferably 50 mM.

In a case of using a cationic buffer solution as the cationic substance, a pH of the cationic buffer solution is not particularly limited as long as the pH of the cationic buffer solution does not have an adverse effect on the growth of the cells and the formation of the cell aggregate. The pH of the cationic buffer solution used in the present embodiment is preferably from 6.0 to 8.0. For example, the pH of the cationic buffer solution used in the present embodiment is 7.0, 7.1, 7.2, 7.3, 7.4, 7.5, 7.6, 7.7, 7.8, 7.9, or 8.0. The pH of the cationic buffer solution used in the present embodiment is more preferably from 7.2 to 7.6. The pH of the cationic buffer solution used in the present embodiment is further more preferably 7.4.

In the present specification, the "polymeric electrolyte" means a polymer having a dissociable functional group in a polymer chain as defined in claim 1.

The polymeric electrolyte may be used alone, or may be used in combination. The polymeric electrolyte used in the present embodiment is preferably glycosaminoglycan. In addition, the polymeric electrolyte used in the present embodiment is more preferably heparin or dextran sulfate, chondroitin sulfate, or dermatan sulfate. The polymeric electrolyte used in the present embodiment is further more preferably heparin.

A concentration of the polymeric electrolyte is more than 0 mg/mL and less than 1.0 mg/mL. The concentration of the polymeric electrolyte used in the present embodiment is preferably 0.025 mg/mL or more to 0.1 mg/mL or less. For example, the concentration of the polymeric electrolyte used in the present embodiment is 0.025, 0.05, 0.075, or 0.1 mg/mL. The concentration of the polymeric electrolyte used in the present embodiment is further more preferably 0.05 mg/mL or more to 0.1 mg/mL or less. The concentration of the polymeric electrolyte used in the present embodiment is further more preferably 0.05 mg/mL. In the present embodiment, the polymeric electrolyte may be appropriately dissolved in a solvent and used. Examples of the solvent include water and a buffer solution, but are not limited thereto. In a case of using a cationic buffer solution as the cationic substance, the polymeric electrolyte may be dissolved in the cationic buffer solution and used.

The extracellular matrix component used in the present embodiment is selected from the group consisting of collagen, laminin, fibronectin, vitronectin, elastin, tenascin, entactin, fibrillin, proteoglycan, and a combination thereof. Examples of the proteoglycan include chondroitin sulfate proteoglycan, heparan sulfate proteoglycan, keratan sulfate proteoglycan, and dermatan sulfate proteoglycan, but are not limited thereto. The extracellular matrix component used in the present embodiment is collagen, laminin, and fibronectin, and among these, collagen is preferable.

A concentration of the extracellular matrix component is more than 0 mg/mL and less than 1.0 mg/mL. The concentration of the extracellular matrix component used in the present embodiment is preferably 0.025 mg/mL or more to 0.1 mg/mL or less. For example, the concentration of the extracellular matrix component used in the present embodiment is 0.025, 0.05, 0.075, or 0.1 mg/mL. The concentration of the extracellular matrix component used in the present embodiment is more preferably 0.05 mg/mL or more to 0.1 mg/mL or less. The concentration of the extracellular matrix component used in the present embodiment is further more preferably 0.05 mg/mL. In the present embodiment, the extracellular matrix component may be dissolved in a solvent and used. Examples of the solvent include water, a buffer solution, and acetate, but are not limited thereto. In the present embodiment, the extracellular matrix component is preferably dissolved in the buffer solution or acetate.

In the present embodiment, a mixture ratio of the polymeric electrolyte to the extracellular matrix component is preferably 1:2 to 2:1. The mixture ratio of the polymeric electrolyte to the extracellular matrix component used in the present embodiment is more preferably 1:1.5 to 1.5:1. The mixture ratio of the polymeric electrolyte to the extracellular matrix component used in the present embodiment is further more preferably 1:1.

Cells used in the method according to the present embodiment are not particularly limited. For example, the cells are cells derived from animals such as humans, monkeys, dogs, cats, rabbits, pigs, cows, mice, and rats. Cell-derived sites are also not particularly limited, and may be somatic cells derived from bones, muscles, viscera, nerves, brain, skin, blood, and the like, or may be reproductive cells. In addition, the cells used in the method according to the present embodiment may be induced pluripotent stem cells (iPS cells) or embryonic stem cells (ES cells). Or, the cells used in the method according to the present embodiment may be culture cells such as primary culture cells, subculture cells, and cell strain cells. One kind of cell may be used, or a plurality of kinds of cells may be used. Examples of the cells used in the method according to the present embodiment include nerve cells, dendritic cells, immune cells, vascular endothelial cells, lymphatic endothelial cells, fibroblasts, cancer cells such as hepatoma cells, epithelial cells, myocardial cells, liver cells, pancreatic islet cells, tissue stem cells, and smooth muscle cells, but are not limited thereto.

The three-dimensional cell tissue obtained by the method according to the present embodiment may include one kind of cell, or may include a plurality of kinds of cells. In the present embodiment, cells included in the three-dimensional cell tissue are selected from the group consisting of nerve cells, dendritic cells, immune cells, vascular endothelial cells, lymphatic endothelial cells, fibroblasts, cancer cells, epithelial cells, myocardial cells, liver cells, pancreatic islet cells, tissue stem cells, and smooth muscle cells. In addition, the three-dimensional cell tissue obtained by the method according to the present embodiment may have a vasculature. The "vasculature" indicates a network-like structure such as vascular network and lymphatic network in tissues of a living organism.

In the method according to the present embodiment, as means for removing a liquid portion in the step (A'-1), a method known to those skilled in the art may be used. For example, a liquid portion may be removed by centrifugal separation or filtration. Conditions of the centrifugal separation are not particularly limited as long as the conditions of the centrifugal separation do not have an adverse effect on the growth of the cells and the formation of the cell aggregate. For example, a liquid portion and a cell aggregate are separated by applying centrifugal separation to a microtube into which a mixture is introduced at room temperature for one minute at 400 × g to remove the liquid portion. Alternatively, the liquid portion may be removed after gathering cells by natural sedimentation.

The solution used in the step (A'-2) of the method according to the present embodiment is not particularly limited as long as the solution used in the step (A'-2) does not have an adverse effect on the growth of the cells and the formation of the cell aggregate. For example, a cell culture medium or a buffer solution suitable for the used cells is used.

Examples of a substrate used in the step (B) or the step (B') of the method according to the present embodiment include a culture vessel used for culture of cells. The culture vessel is a vessel made of a material and having a shape generally used for culture of cells or microorganisms. Examples of the material of the culture vessel include glass, stainless steel, and plastic, but are not limited thereto. Examples of the culture vessel include a dish, a tube, a flask, a bottle, and a plate, but are not limited thereto. For example, the substrate is made of a material that does not pass through cells in a liquid but is able to pass through the liquid.

The substrate used in the present embodiment is preferably a permeable membrane. Examples of a vessel including such a permeable membrane include cell culture inserts such as Transwell (registered trademark) insert, Netwell (registered trademark) insert, Falcon (registered trademark) cell culture insert, and Millicell (registered trademark) cell culture insert, but are not limited thereto.

In the method of the present embodiment, as means for gathering cells in the step (B) and the step (B'), a method known to those skilled in the art may be used. For example, cells may be gathered by centrifugal separation, magnetic separation, or filtration. Conditions of the centrifugal separation are not particularly limited as long as the conditions of the centrifugal separation do not have an adverse effect on the growth of the cells and the formation of the cell aggregate. For example, a mixture or suspension is sown into a cell culture insert, and subjected to centrifugal separation at 10°C for one minute for 400 × g to gather the cells. Alternatively, the cells may be gathered by natural sedimentation. In the step (B'), a cell precipitate may be formed on a substrate by removing the liquid portion from the suspension by the centrifugal separation or filtration. Or, the cell precipitate may be formed on the substrate by natural sedimentation. The cell aggregate in the step (B) or the cell precipitate in the step (B') may be in a layer shape.

In the present embodiment, a substance for suppressing deformation (for example, contraction of a tissue, peeling-off of a tissue terminal, and the like) of the constructed three-dimensional cell tissue is used. Examples of such a substance include Y-27632 which is a selective ROCK (Rho-associated coiled-coil forming kinase/Rho bond kinase) inhibitor, but are not particularly limited thereto. In the present embodiment, a step (C) is performed in the presence of the substance. By culturing a cell aggregate in the presence of the substance, contraction of the constructed three-dimensional cell tissue is suppressed. As a result, peeling-off of the constructed tissue from a substrate such as cell culture insert is suppressed. For example, in the step (A), such a substance may be further mixed. Alternatively, such a substance may be further added to the solution used in the step (A'-2). Alternatively, such a substance may be added to a culture medium when culturing cells in the step (C).

In the step (C) of the method according to the present embodiment, culture of cells may be performed in culture conditions suitable for cultured cells. Those skilled in the art may select an appropriate culture medium depending on the kind of the cells or desired functions. In addition, all conditions such as culture temperature and culture time are easily determined by those skilled in the art.

By repeating the steps (A) to (B) or (A) to (B') of the method of the present embodiment, it is possible to stack cell aggregates or cell precipitates. In this manner, it is possible to construct a three-dimensional cell tissue having a plurality of layers. In this case, a three-dimensional cell tissue constituted of different kinds of cells by using the plurality of kinds of cells. In this embodiment, a thickness of the constructed three-dimensional cell tissue is from about 5 to about 300 µm, about 5 to about 400 µm, or about 5 to about 500 µm. The thickness of the constructed three-dimensional cell tissue is preferably 150 µm or more, more preferably 200 µm or more, and further more preferably 250 µm or more. By repeating the steps (A) to (B) or (A) to (B') of the method of the present invention, a three-dimensional cell tissue having a small amount of voids inside, although having the thickness of from 150 to 500 µm, is obtained. In the embodiment, the number of the cell layers in the constructed three-dimensional cell tissue is preferably from 1 to about 100 layers, and more preferably from 10 to about 100 layers. The cell layer is defined as a separate layer at the time when a cell nucleus does not overlap with gravity and a height direction of the cell, when observed at a magnification at which the cell nucleus can be recognized, that is, at a magnification at which an entire thickness of a stained slice is within a visual field, in a slice image of a cross-section in a thickness direction of the three-dimensional cell tissue. At this time, a visual field in a vertical direction to gravity (horizontal direction) is secured by at least 200 µm or greater. In the present embodiment, a slice image of a cross-section in a thickness direction of the three-dimensional cell tissue is observed at a magnification of 100 to 200 times.

The three-dimensional cell tissue obtained by repeating the steps (A) to (B) or (A) to (B') of the method of the present embodiment has the smaller number of cell layers per unit thickness compared to the three-dimensional cell tissue obtained by a method for stacking cells in the related art. In the present embodiment, the constructed three-dimensional cell tissue is obtained in vitro. In addition, the three-dimensional cell tissue according to the present embodiment contains a cell and an extracellular matrix component, and the number of the cell layers per 10 µm of thickness of the three-dimensional cell tissue is 2.8 layers or less, preferably 2.5 layers or less, and more preferably 2.2 layers or less. In the present embodiment, in the obtained three-dimensional cell tissue, the number of cells per area of 100 µm in a thickness direction and 50 µm in a width direction in a region including a position (maximum point) at which the thickness becomes maximum is preferably from 5 to 70, more preferably from 10 to 60, and further more preferably from 15 to 50.

In the present specification, the thickness of the three-dimensional cell tissue is a length of the tissue in the gravity direction. The gravity direction is a direction in which gravity is imparted. In the present embodiment, it is possible to measure the number of the cells as follows by using a slice formed from a cross-section in the thickness direction of the tissue. In the slice, among a region in which there is no void with a predetermined size or greater, a position (maximum point) at which the thickness of the three-dimensional cell tissue is maximum is determined. By setting a strip-like region (including a top surface to a bottom surface of the tissue) of a width of 50 µm including vicinity of the maximum value of the thickness to a measurement region, the number of the cell nuclei included in the measurement region is counted. The measurement region is set not to include a void. The predetermined size of the void is set to be a maximum diameter of 50 µm or greater. The maximum diameter of the void is defined as a long side in a case where the void is rectangular, is defined as a diameter in a case where the void is spherical, is defined as a long diameter in a case where the void is elliptic, and is defined as a long diameter of an approximated ellipse in a case where the void is amorphous. The void is not stained on an HE-stained slice. Here, in a case where the top face of the region including the maximum value of the thickness is in a convex shape, there is a case where the tissue is peeled off from the substrate. In such a case, a region which is vicinity of the maximum value and of which the top surface is relatively plain is set as a measurement region. In this case, a region of 100 µm to 650 µm in a width direction including the vicinity of the maximum value of the thickness is set as the measurement region.

A cell nucleus of which at least a portion is included in the measurement region to which counting is performed is counted as a cell nucleus included in the measurement region. From the counted cells, a number of the cells per area of 100 µm in the thickness direction and 50 µm in the width direction is calculated.

A kit used according to one embodiment of the present invention is a kit for preforming the method according to the embodiment, and includes at least one reagent selected from a cell, a cationic substance, a polymeric electrolyte, and an extracellular matrix component as defined in claim 13. in general, the reagent is put in an appropriate container and provided. Such a kit may include a reagent suitable for conveniently performing the method of the embodiment, for example, a diluent, a buffer, and a washing reagent. In addition, the kit may include a suitable substrate such as dish, cell culture insert, tube, flask, bottle, and plate. Moreover, the kit may include materials such as instruction necessary for carrying out the method of the embodiment.

Using the method according to the embodiment of the present invention, the number of times to subject cells to centrifugal separation is in two. In the methods in the related art, the operation is complex and the centrifugal separation is also used a lot of times. Therefore, by using the method according to the embodiment of the present invention, it is possible to reduce the number of times of the centrifugal separation and to decrease damage of the cells. In addition, it is possible to reduce loss of the cells during operation and to achieve a high cell recovery rate.

Hereinafter, a detailed and specific description will be provided on the present invention by showing examples. However, examples do not limit the scope of the present invention.

### Examples

### Example 1. Construction of Three-dimensional Cell Tissue using Heparin and Collagen (1)

In the following example, collagen I was used as collagen, unless otherwise explained.

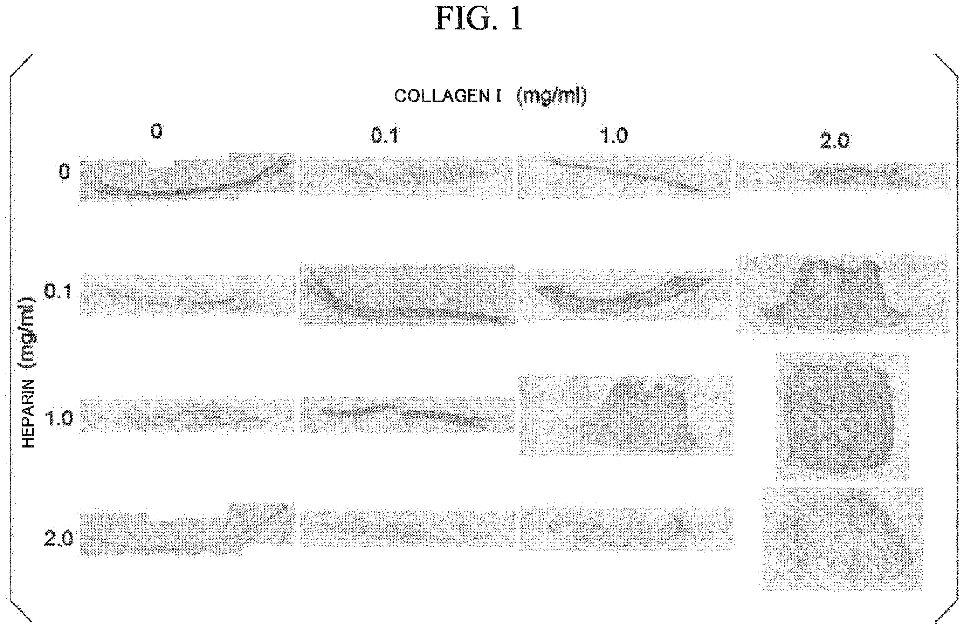

Normal human dermal fibroblast (NHDF) cells of 3.5 × 10⁶ cells were suspended in a mixture solution of 150 µL of a solution of heparin/50 mM tris-hydrochloric acid buffer solution (pH 7.4) and 150 µL of collagen/50 mM tris-hydrochloric acid buffer solution (pH 7.4). Combination of final concentrations of the used heparin and collagen is as shown in FIG. 1. The obtained suspension was sown into a 24 well cell culture insert (Corning Inc, Catalog number: 3470), and centrifuged at 10°C for one minute at 400 × g. Accordingly, a cell layer was formed on the cell culture insert. Subsequently, a Dulbecco's modified eagle's medium (DMEM) containing 10% fetal bovine serum (FBS) was added to the cell culture insert, and culture was performed in a CO₂ incubator (37°C, 5% CO₂) for 24 hours. After the culture, a constructed tissue was collected, and a paraffin embedding slice was prepared. Preparation of the paraffin embedding slice was performed by a known method. The prepared slice was subjected to hematoxylin eosin stain (HE stain). The HE stain was performed by a known method.

The result of the HE stain is shown in FIG. 1. In a case of heparin alone and collagen alone, formation of a tissue was not shown. In a case where a concentration of heparin was 1.0 mg/mL or more and a concentration of collagen was 1.0 mg/mL or more, formation of aggregation of collagen and contraction of a tissue were observed. From the result, it was shown that, in a case of using NHDF, both of the concentration of heparin and the concentration of collagen were preferably more than 0 mg/mL and less than 1.0 mg/mL. The formation of aggregation of collagen was improved by using a collagen/acetic acid solution (pH 3.7) instead of a solution of collagen/50 mM tris-hydrochloric acid buffer (pH 7.4) solution. In addition, even in a case where a suspension of heparin, collagen, and cells was sown into the cell culture insert, and then a cell layer was formed by natural sedimentation without performing centrifugal operation, it was possible to construct a three-dimensional cell tissue.

### Example 2. Construction of Three-dimensional Cell Tissue using Heparin and Collagen (2)

NHDF cells of 3.5 × 10⁶ cells were suspended in a mixture solution of 250 µL of a solution of 0.1 mg/mL of heparin/50 mM tris-hydrochloric acid buffer solution (pH 7.4) and 250 µL of 0.1 mg/mL of collagen/acetic acid solution (pH 3.7) (that is, each of the final concentrations of collagen and heparin was 0.05 mg/mL). Sample A and Sample B were prepared as follows.

### (i) Sample A

The obtained mixture was sown into a 24 well cell culture insert, and centrifuged at 10°C for one minute at 400 × g. Accordingly, a cell layer was formed on the cell culture insert. Subsequently, a DMEM containing 10% FBS was added to the cell culture insert, and culture was performed in a CO₂ incubator (37°C, 5% CO₂) for 24 hours. After the culture, a constructed tissue was collected, and a paraffin embedding slice was prepared. Preparation of the paraffin embedding slice was performed by a known method. The prepared slice was subjected to HE stain. The HE stain was performed by a known method.

### (ii) Sample B

The obtained mixture was centrifuged at room temperature for one minute at 400 × g to obtain a viscous body. The obtained viscous body was suspended in a DMEM containing 10% FBS. The obtained suspension was sown into a 24 well cell culture insert, and centrifuged at 10°C for one minute at 400 × g (gravitational acceleration). Accordingly, a cell layer was formed on the cell culture insert. Subsequently, a DMEM containing 10% FBS was added to the cell culture insert, and culture was performed in a CO₂ incubator (37°C, 5% CO₂) for 24 hours. After the culture, a constructed tissue was collected, and a paraffin embedding slice was prepared. Preparation of the paraffin embedding slice was performed by a known method. The prepared slice was subjected to HE stain. The HE stain was performed by a known method.

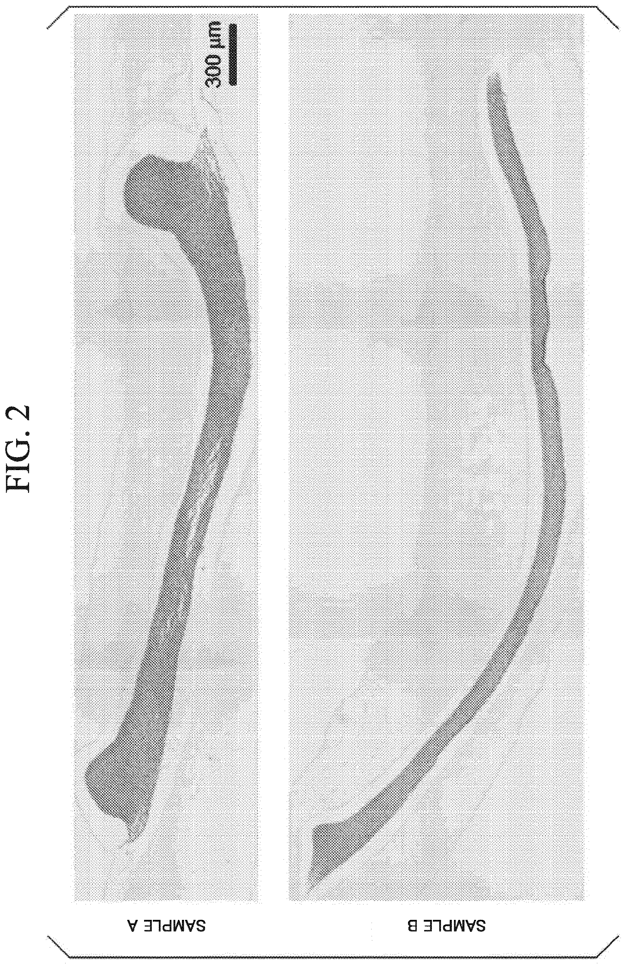

The result of the HE stain is shown in FIG. 2. In Sample A, an end of the constructed tissue was peeled off from a surface of the cell culture insert. Dissociation of the tissue in the vicinity of the center was also slightly observed but a three-dimensional cell tissue was obtained as a whole. In Sample B, a more homogeneous three-dimensional cell tissue was obtained compared to Sample A. From the result, it was shown that, before sowing a mixture of cells, heparin, and collagen into a culture container, by obtaining a viscous body from the mixture, and using the viscous body, it was possible to obtain a more homogeneous three-dimensional cell tissue. In addition, regarding Sample B, even in a case where a suspension of the viscous body was sown into the cell culture insert, and then a cell layer was formed by natural sedimentation without performing centrifugal operation, it was possible to construct a three-dimensional cell tissue.

### Example 3. Additional Examination of Concentrations of Heparin and Collagen

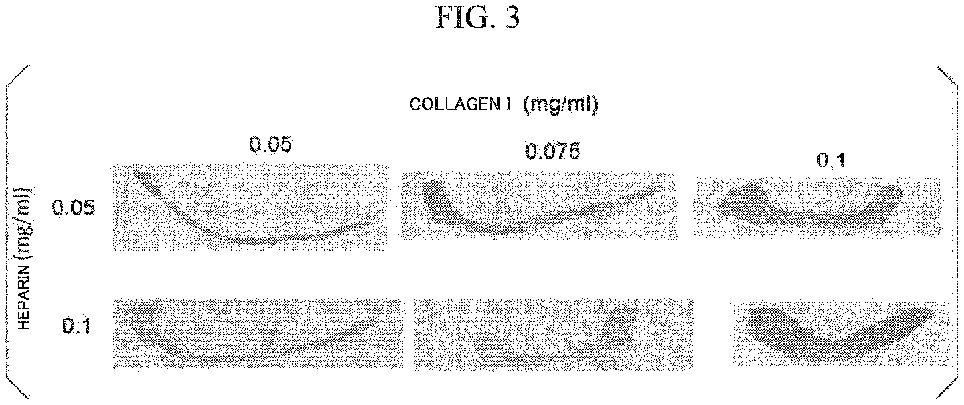

NHDF of 3.5 × 10⁶ cells were suspended in a mixture solution of 250 µL of heparin/ 50 mM tris-hydrochloric acid buffer solution (pH 7.4), and 250 µL of collagen/ acetic acid solution (pH 3.7). Combination of final concentrations of the used heparin and collagen is as shown in FIG. 3. The obtained mixture was centrifuged at room temperature for one minute at 400 × g to obtain a viscous body. The obtained viscous body was suspended in a DMEM containing 10% FBS. The obtained suspension was sown into a 24 well cell culture insert, and centrifuged at 10°C for one minute at 400 × g (gravitational acceleration). Accordingly, a cell layer was formed on the cell culture insert. Subsequently, a DMEM containing 10% FBS was added to the cell culture insert, and culture was performed in a CO₂ incubator (37°C, 5% CO₂) for 24 hours. After the culture, a constructed tissue was collected, and a paraffin embedding slice was prepared.

Preparation of the paraffin embedding slice was performed by a known method. The prepared slice was subjected to HE stain. The HE stain was performed by a known method.

The result of the HE stain is shown in FIG. 3. In a case where a final concentration of collagen was 0.075 mg/mL or more, peeling-off of both ends of the constructed tissue from a surface of the cell culture insert was prominently observed.

### Example 4. Construction of Continuous Stacked Tissue

Using NHDF previously subjected to fluorescent stain with cell tracker green and NHDF previously subjected to fluorescent stain with cell tracker red, a continuous stacked tissue was constructed. The procedure of construction of a tissue is as follows.

### (i) Three-layer tissue

NHDF previously subjected to fluorescent stain with cell tracker red of 1.0 × 10⁶ cells were suspended in a mixture solution of 150 µL of a solution of 0.2 mg/mL of heparin/50 mM tris-hydrochloric acid buffer solution (pH 7.4) and 150 µL of 0.2 mg/mL of collagen/acetic acid solution (pH 3.7) (that is, each of the final concentrations of collagen and heparin was 0.1 mg/mL). Following the procedure of (ii) of Example 2, a first cell layer was formed on a cell culture insert. By the same procedure, using NHDF previously subjected to fluorescent stain with cell tracker green of 1.0 × 10⁶ cells, a second cell layer was formed on the first cell layer. In addition, by the same procedure, using NHDF previously subjected to fluorescent stain with cell tracker red of 1.0 × 10⁶ cells, a third cell layer was formed on the second cell layer. Subsequently, a DMEM containing 10% FBS was added to the cell culture insert, and culture was performed in a CO₂ incubator (37°C, 5% CO₂) for 24 hours. After the culture, a constructed tissue was collected, and a frozen slice was prepared. Preparation of the frozen slice was performed by a known method.

### (ii) Five-layer tissue

NHDF previously subjected to fluorescent stain with cell tracker green of 0.6 × 10⁶ cells were suspended in a mixture solution of 150 µL of a solution of 0.2 mg/mL of heparin/50 mM tris-hydrochloric acid buffer solution (pH 7.4) and 150 µL of 0.2 mg/mL of collagen/acetic acid solution (pH 3.7). Following the procedure of (ii) of Example 2, a first cell layer was formed on a cell culture insert. By the same procedure, using NHDF previously subjected to fluorescent stain with cell tracker red of 0.6 × 10⁶ cells, a second cell layer was formed on the first cell layer. In addition, by the same procedure, using NHDF previously subjected to fluorescent stain with cell tracker green of 0.6 × 10⁶ cells, a third cell layer was formed on the second cell layer. Furthermore, by the same procedure, using NHDF previously subjected to fluorescent stain with cell tracker red of 0.6 × 10⁶ cells, a fourth cell layer was formed on the third cell layer. Moreover, by the same procedure, using NHDF previously subjected to fluorescent stain with cell tracker green of 0.6 × 10⁶ cells, a fifth cell layer was formed on the fourth cell layer. Subsequently, a DMEM containing 10% FBS was added to the cell culture insert, and culture was performed in a CO₂ incubator (37°C, 5% CO₂) for 24 hours. After the culture, a constructed tissue was collected, and a frozen slice was prepared. Preparation of the frozen slice was performed by a known method.

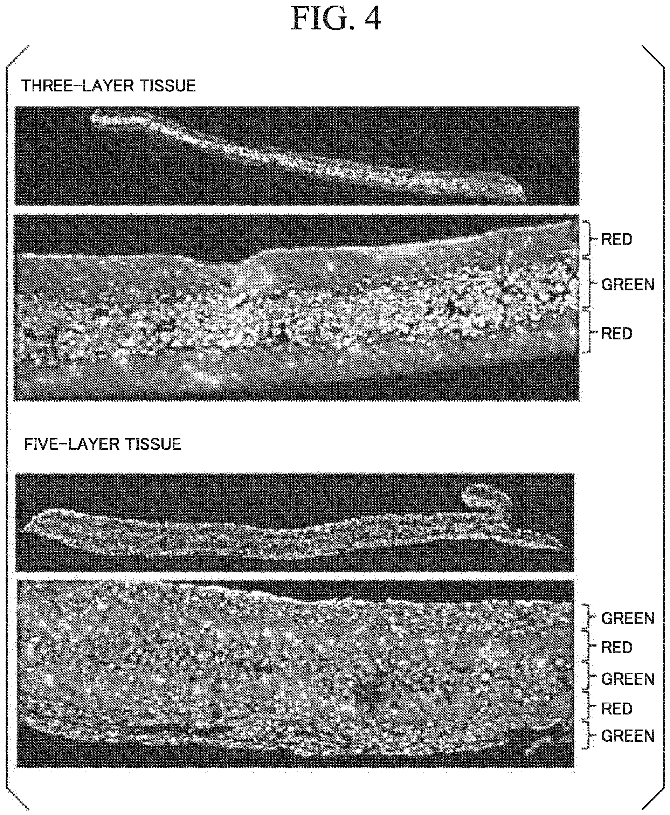

The result of fluorescence microscope observation of the slice is shown in FIG. 4. The upper figure is a view of the entire tissue, and the lower figure is an enlarged view thereof, respectively. A layer structure can be observed in the constructed tissue.

### Example 5. Construction of Cancer Tissue Model

Using NHDF and colorectal cancer cells (HT29 cells), a cancer tissue model was constructed. NHDF were previously subjected to fluorescent stain with cell tracker green. In addition, HT29 cells were previously subjected to fluorescent stain with cell tracker red. 3.5 × 10⁶ cells (90% NHDF, 10% HT29 cells) were suspended in a mixture solution of 250 µL of a solution of 0.1 mg/mL of heparin/50 mM tris-hydrochloric acid buffer solution (pH 7.4) and 250 µL of 0.1 mg/mL of collagen/acetic acid solution (pH 3.7) (that is, each of final concentrations of heparin and collagen was 0.05 mg/mL). Following the procedure of (ii) of Example 2, a cell layer was formed on a cell culture insert. Subsequently, a DMEM containing 10% FBS was added to the cell culture insert, and culture was performed in a CO₂ incubator (37°C, 5% CO₂) for 24 hours.

After the culture, a constructed tissue was collected, and a frozen slice was prepared. Preparation of the frozen slice was performed by a known method. The prepared slice was subjected to HE stain. The HE stain was performed by a known method.

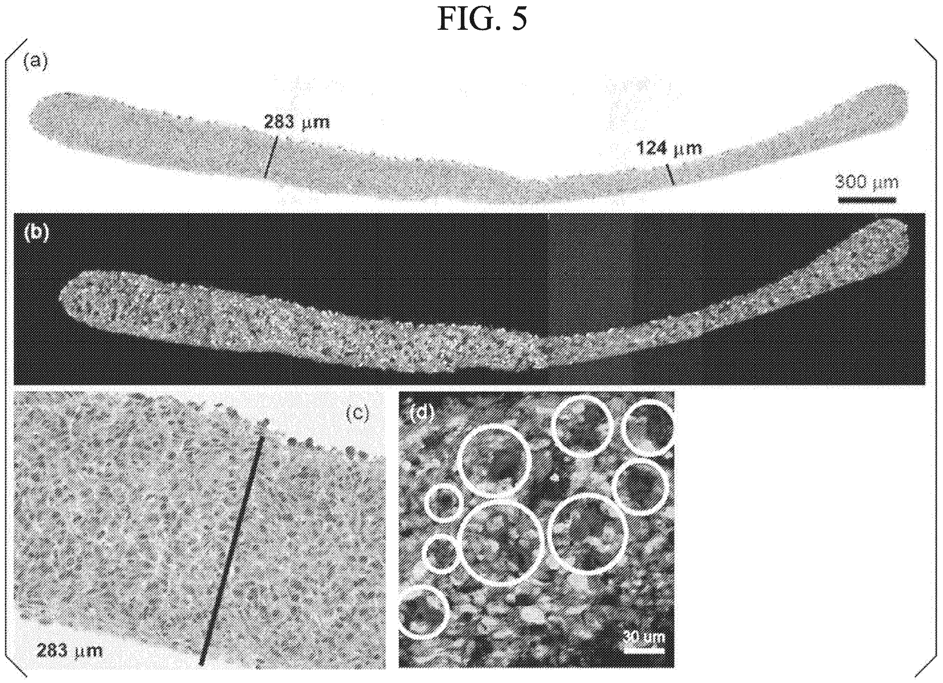

The result of the HE stain and the result of fluorescence microscope observation are shown in FIG. 5. FIG. 5(a) shows a result of the HE stain of the frozen slice. FIG. 5(b) shows a result of fluorescence microscope observation of the frozen slice. FIG. 5(c) is an enlarged view of FIG. 5(a). FIG. 5(d) is an enlarged view of FIG. 5(b). In FIG. 5(d), in an area surrounded by a white circle, HT29 cells stained with red color were observed.

### Example 6. Suppression of Contraction of Tissue

Using Y-27632 (Calbiochem, Catalog number: 688000), suppression of contraction of the constructed tissue was examined. In the present example, concentrations of heparin and collagen, at which peeling-off of both ends of the tissue from a surface of the cell culture insert was observed in Example 3 (FIG. 3) were employed. NHDF were suspended in a mixture solution of 250 µL of a solution of 0.2 mg/mL of heparin/50 mM tris-hydrochloric acid buffer solution (pH 7.4) and 250 µL of 0.2 mg/mL of collagen/acetic acid solution (pH 3.7) (that is, each of the final concentrations of collagen and heparin was 0.1 mg/mL). Following the procedure of (ii) of Example 2, a cell layer was formed on a cell culture insert. The number of used cells and the solution used for suspension of a viscous body are as shown in the following table.

**[Table 1]**

| | Number of cells | Solution used for suspension |
|---|---|---|
| Sample (a) | 3.5×10⁶ | DMEM containing 10% FBS |
| Sample (b) | 3.5×10⁶ | DMEM containing 10% FBS (5 mM Y-27632 added) |
| Sample (c) | 5.0×10⁶ | DMEM containing 10% FBS (5 mM Y-27632 added) |

Subsequently, a DMEM containing 10% FBS was added to the cell culture insert, and culture was performed in a CO₂ incubator (37°C, 5% CO₂) for 24 hours. After the culture, a constructed tissue was collected, and a paraffin embedding slice was prepared. Preparation of the paraffin embedding slice was performed by a known method. The prepared slice was subjected to HE stain. The HE stain was performed by a known method.

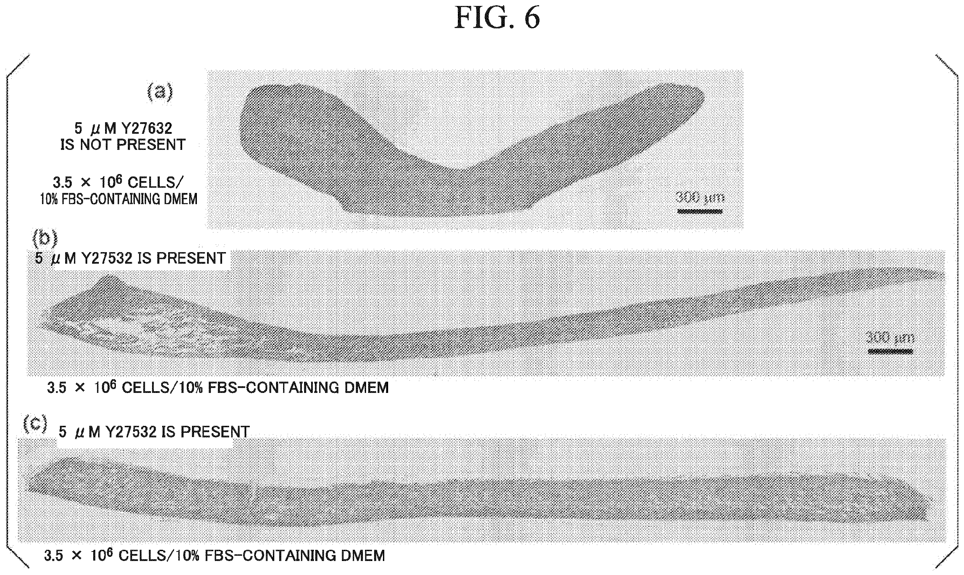

The result of the HE stain is shown in FIG. 6. By adding Y-27632, contraction of the constructed tissue was suppressed, and thus more homogeneous tissue was obtained. In addition, even in a case of using 5.0 × 10⁶ cells, both ends of the tissue were not peeled off from a surface of the cell culture insert (substrate), and it was possible to obtain a more homogeneous tissue. The average thickness of the tissue was 270 µm.

### Example 7. Construction of Three-dimensional Cell Tissue Having Vasculature (1)

NHDF of 2.0 × 10⁶ cells and human umbilical vein endothelial cells (HUVEC) of 3.0 × 10⁴ cells were suspended in a mixture solution of 150 µL of a solution of 0.2 mg/mL of heparin/50 mM tris-hydrochloric acid buffer solution (pH 7.4) and 150 µL of 0.2 mg/mL of collagen/acetic acid solution (pH 3.7) (that is, each of the final concentrations of heparin and collagen was 0.1 mg/mL), and then centrifuged at room temperature for one minute at 400 × g to obtain a viscous body. After the obtained viscous body was suspended in a DMEM containing 10% FBS, the obtained suspension was sown into a 24 well cell culture insert, and centrifuged at room temperature for one minute at 400 × g. Subsequently, a DMEM containing 10% FBS was added to the cell culture insert, and culture was performed in a CO₂ incubator (37°C, 5% CO₂) for eight days. After the culture, using an anti-CD31 antibody (monoclonal mouse anti-human CD31 antibody, Clone JC70A, Code M0823, Dako) as a primary antibody, and goat anti-mouse IgG-AlexaFluor (registered trademark) 488 (invitrogen (trademark)) as a secondary antibody, the constructed tissue was subjected to immune stain. The immune stain was performed by a known method.

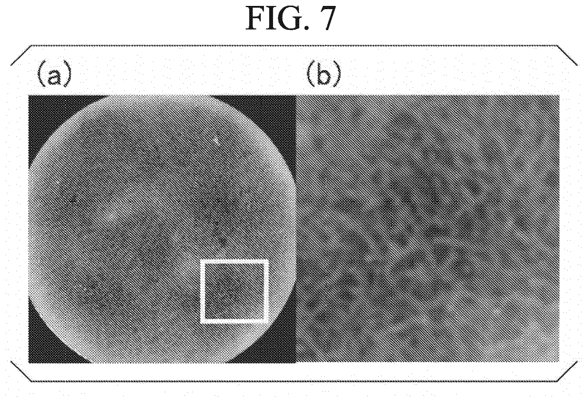

The result of the immune stain is shown in FIG. 7. As shown in FIG. 7, a vascular network was formed in the tissue.

### Example 8. Construction of Three-dimensional Cell Tissue Having Vasculature (2)

NHDF of 1.0 × 10⁶ cells and human umbilical vein endothelial cells (HUVEC) of 1.0 × 10⁵ cells were suspended in a mixture solution of 250 µL of a solution of 0.1 mg/mL of heparin/50 mM tris-hydrochloric acid buffer solution (pH 7.4) and 250 µL of 0.1 mg/mL of collagen/acetic acid solution (pH 3.7) (that is, each of the final concentrations of heparin and collagen was 0.05 mg/mL). Following the procedure of (ii) of Example 2, a cell layer was formed on a cell culture insert. Subsequently, a DMEM containing 10% FBS was added to the cell culture insert, and culture was performed in a CO₂ incubator (37°C, 5% CO₂) for three days.

After the culture, the constructed tissue was collected, and a paraffin embedding slice was prepared. Preparation of the paraffin embedding slice was performed by a known method. The prepared slice was subjected to HE stain. The HE stain was performed by a known method. In addition, using an anti-CD31 antibody (monoclonal mouse anti-human CD31 antibody, Clone JC70A, Code M0823, Dako), immune stain was performed.

The immune stain was performed by a known method. 3,3'-Diaminobenzidine (DAB) was used as a chromogenic substrate, and hematoxylin was used as a nuclei staining reagent.

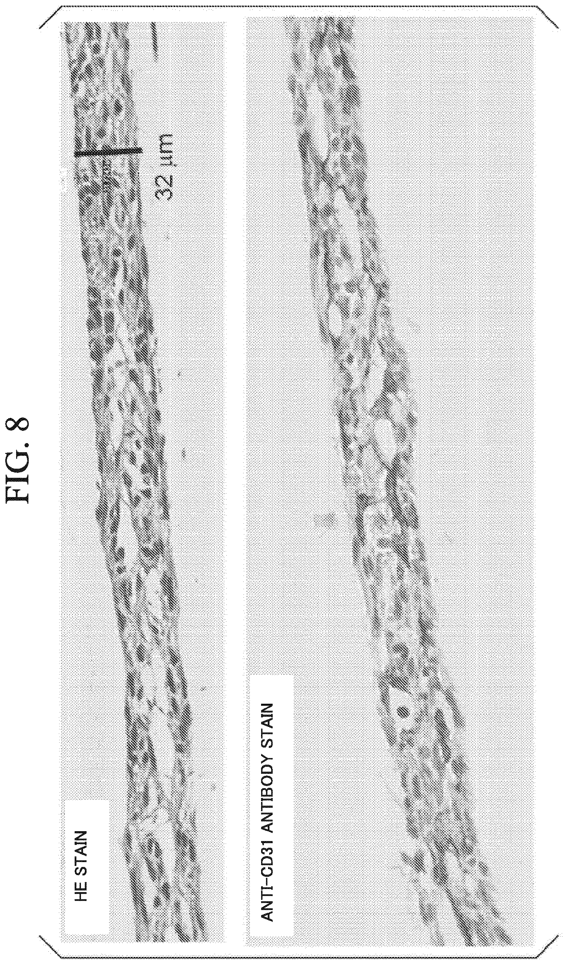

The result of the HE stain is shown in FIG. 8. As shown in FIG. 8, a vasculature surrounded by CD31 positive cells was observed in the tissue. From the results shown in FIGS. 7 and 8, it was shown that, by using fibroblast cells and vascular endothelial cells in combination, it was possible to obtain a three-dimensional cell tissue having a vascular network.

### Comparative Example 1. Case of Not Using Heparin and Collagen

NHDF of 3.5 × 10⁶ cells suspended in a DMEM containing 10% FBS were sown into a 24 well cell culture insert, and culture was performed in a performed in a CO₂ incubator (37°C, 5% CO₂) for 24 hours. After the culture, the constructed tissue was collected, and a paraffin embedding slice was prepared. Preparation of the paraffin embedding slice was performed by a known method. The prepared slice was subjected to HE stain. The HE stain was performed by a known method.

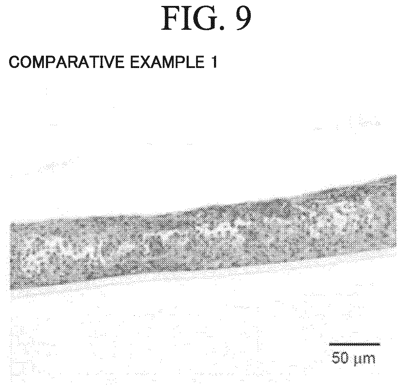

The result of the HE stain of Comparative Example 1 is shown in FIG. 9. In Comparative Example 1, only a tissue thinner than that in examples was constructed. In addition, dissociation was observed in a central portion of the tissue.

### Comparative Example 2. Case of Using the Method Disclosed in Patent Document 5

In a case of employing the method disclosed in Patent Document 5, it was verified whether or not it is possible to construct a three-dimensional cell tissue. A culture solution of NHDF was centrifuged using a microtube, and a supernatant was removed to recover cells. The recovered cells were mixed with a 0.1% collagen solution (dissolved in a DMEM containing 10% FBS), subjected to rotary stirring at 4°C for 10 minutes to obtain a suspension of the cells. The number of the used cells was 1 × 10⁵ cells, 1 × 10⁶ cells, and 3.5 × 10⁶ cells. The cell suspension was centrifuged at room temperature for 1 minute at 400 × g, and a supernatant was removed to obtain a cell aggregate. The cell aggregate was sown into a cell culture insert, and centrifuged at room temperature for 1 minute at 400 × g. Subsequently, a DMEM containing 10% FBS was added to the cell culture insert, and culture was performed in a CO₂ incubator (37°C, 5% CO₂) for 24 hours. After the culture, the constructed tissue was collected, and a paraffin embedding slice was prepared. Preparation of the paraffin embedding slice was performed by a known method. The prepared slice was subjected to HE stain. The HE stain was performed by a known method.

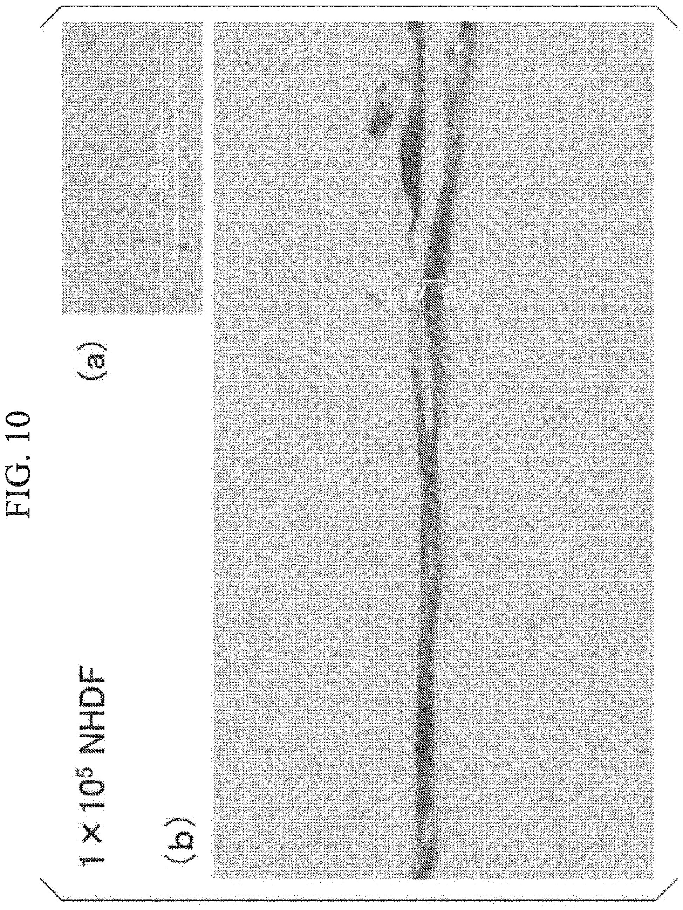

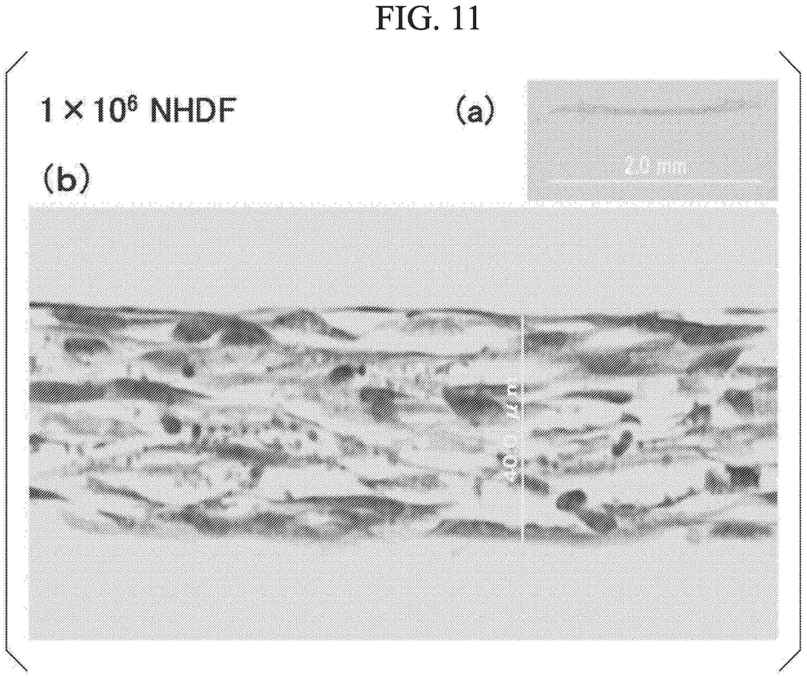

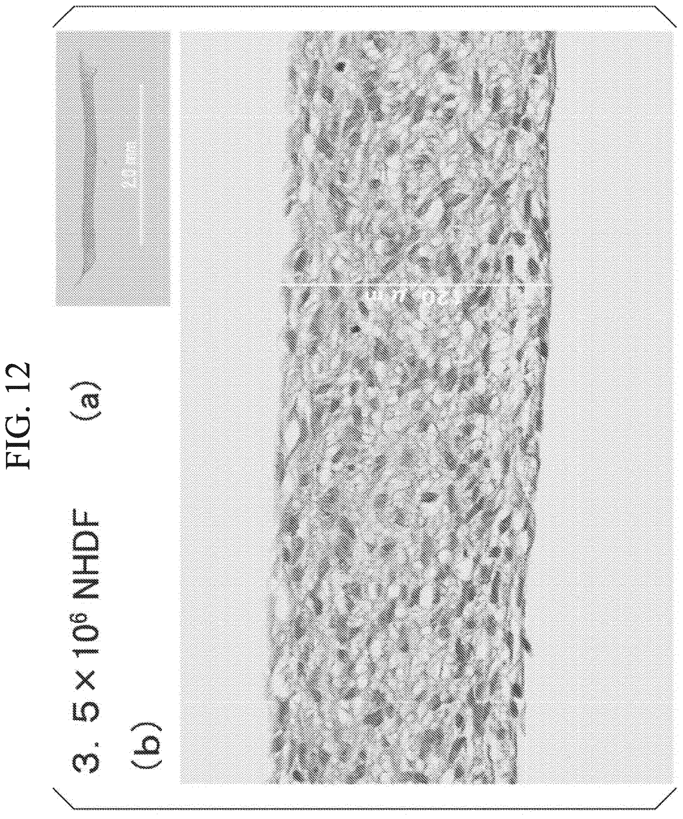

The result of the HE stain of Comparative Example 2 is shown in FIGS. 10 to 12. In each figure, (a) shows an entire view of the obtained tissue, and (b) is an enlarged view thereof. FIG. 10 shows a result obtained by using NHDF of 1 × 10⁵ cells. A thickness of the obtained tissue is about 5 µm, and it was not possible to discern each cell in the tissue. FIG. 11 shows a result obtained by using NHDF of 1 × 10⁶ cells. A thickness of the obtained tissue was about 40 µm, but in the tissue, a lot of spaces were seen between cells. FIG. 12 shows a result obtained by using NHDF of 3.5 × 10⁶ cells. A thickness of the obtained tissue was about 120 µm, but in the tissue, a lot of spaces were seen between cells. In other words, although the method disclosed in Patent Document 5 was employed, it was not possible to obtain a desired three-dimensional cell tissue.

### Comparative Example 3. LbL Method

According to the method (LbL method) for producing a three-dimensional tissue disclosed in Patent Document 10, NHDF of 3.5 × 10⁶ cells in which fibronectin-gelatin thin membrane (FN-G thin membrane) was formed were sown into a cell culture insert. Subsequently, a DMEM containing 10% FBS was added to the cell culture insert, and culture was performed in a CO₂ incubator (37°C, 5% CO₂) for 24 hours. After the culture, the constructed tissue was collected, and a paraffin embedding slice was prepared. Preparation of the paraffin embedding slice was performed by a known method. The prepared slice was subjected to HE stain. The HE stain was performed by a known method.

In Comparative Example 3, dissociation was seen in a central portion of the obtained tissue. That is, by using cells of 3.5 × 10⁶ cells or greater, it was not possible to form a tissue with a thickness more than 100 µm.

### Example 9. Construction of Three-dimensional Cell Tissue Using Various Cationic Substances

Using tris, bistris, and HEPES as a cationic substance, heparin, and collagen was mixed with cells to construct a three-dimensional cell tissue.

NHDF of 3.5 × 10⁶ cells were suspended in a mixture solution of 250 µL of a solution of 0.1 mg/mL of heparin/50 mM tris-hydrochloric acid buffer solution (pH 7.4) and 250 µL of 0.1 mg/mL of collagen/acetic acid solution (pH 3.7) (that is, each of final concentrations of heparin and collagen was 0.05 mg/mL) to prepare a mixture.

Using a solution of 0.1 mg/mL of heparin/50 mM bistris-hydrochloric acid buffer solution (pH 7.4), a solution of 0.1 mg/mL of heparin/50 mM tris-maleic acid buffer solution (pH 7.4), a solution of 0.1 mg/mL of heparin/50 mM HEPES buffer solution (pH 7.4), or 0.1 mg/mL of heparin/DMEM (acetic acid added, pH 7.4 to 7.8), instead of the solution of 0.1 mg/mL of heparin/50 mM tris-hydrochloric acid buffer solution (pH 7.4), a mixture in which each of final concentrations of heparin and collagen was 0.05 mg/mL was prepared in the same manner.

In addition, NHDF of 3.5 × 10⁶ cells were suspended in a mixture solution of 250 µL of a solution of 0.2 mg/mL of heparin/50 mM tris-hydrochloric acid buffer solution (pH 7.4) and 250 µL of 0.2 mg/mL of collagen/acetic acid solution (pH 3.7) (that is, each of final concentrations of heparin and collagen was 0.1 mg/mL) to prepare a mixture.

In addition, using a solution of 0.2 mg/mL of heparin/50 mM bistris-hydrochloric acid buffer solution (pH 7.4), a solution of 0.2 mg/mL of heparin/50 mM tris-maleic acid buffer solution (pH 7.4), a solution of 0.2 mg/mL of heparin/50 mM HEPES buffer solution (pH 7.4), or 0.2 mg/mL of heparin/DMEM (acetic acid added, pH 7.4 to 7.8), instead of the solution of 0.2 mg/mL of heparin/50 mM tris-hydrochloric acid buffer solution (pH 7.4), a mixture in which each of final concentrations of heparin and collagen was 0.1 mg/mL was prepared in the same manner.

For comparison, NHDF of 3.5 × 10⁶ cells were suspended in a mixture solution of 500 µL of a solution of 50 mM tris-hydrochloric acid buffer solution (pH 7.4) to prepare a mixture in which each of final concentration of heparin and collagen was 0 mg/mL. In addition, using a solution of 50 mM bistris-hydrochloric acid buffer solution (pH 7.4), a solution of 50 mM tris-maleic acid buffer solution (pH 7.4), a 50 mM HEPES buffer solution (pH 7.4), or DMEM (acetic acid added, pH 7.4 to 7.8), instead of the solution of 50 mM tris-hydrochloric acid buffer solution (pH 7.4), a mixture in which each of final concentrations of heparin and collagen was 0 mg/mL was prepared in the same manner.

The obtained mixture was centrifuged at room temperature for one minute at 400 × g to obtain a viscous body. The obtained viscous body was suspended in DMEM containing 10% FBS. The obtained suspension was sown into a 24 well cell culture insert, and centrifuged at room temperature for one minute at 400 × g. Accordingly, a cell layer was formed on the cell culture insert. Subsequently, a DMEM containing 10% FBS was added to the cell culture insert, and culture was performed in a CO₂ incubator (37°C, 5% CO₂) for 24 hours. After the culture, a constructed tissue was fixed with 10% formalin, immersed with 70% ethanol, and then a paraffin embedding slice was prepared. Preparation of the paraffin embedding slice was performed by a known method. The prepared slice was subjected to HE stain. The HE stain was performed by a known method.

From each HE-stained evaluation slice, a maximum thickness of each of the constructed three-dimensional cell tissues was measured. The result of measurement is shown in Table 2. As a result, the three-dimensional cell tissue obtained by mixing cells with a cationic substance, heparin, and collagen had a sufficient thickness, and voids were almost not observed inside the tissue, regardless of which cationic substance was used for the tissue. In addition, it was possible to perform culture stably for several days, and there was no problem in culture medium exchange. On the contrary, the three-dimensional cell tissue obtained by mixing cells with DMEM, heparin, and collagen without using a cationic substance had a certain degree of thickness but there existed a lot of small voids inside the tissue. In addition, regarding the tissue obtained by containing only a cationic substance or DMEM without containing heparin and collagen, there existed a lot of small voids inside the tissue, and the tissue itself was fragile. For this reason, the tissue could not stand culture for several days and the cells were collapsed during culture medium exchange.

**[Table 2]**

| Thickness of tissue (mm) | Each of concentrations of heparin and collagen in mixture (mg/mL) | | |
|---|---|---|---|
| | 0.1 | 0.05 | 0 |
| Tris-hydrochloric acid | 3.83 | 5.08 | 2.45 |
| Bistris-hydrochloric acid | 4.48 | 5.83 | 3.15 |
| Tris-maleic acid | 3.80 | 5.09 | 1.83 |
| HEPES | 3.73 | 4.49 | 3.03 |
| DMEM | 2.85 | 4.19 | 2.75 |

### Example 10. Construction of Three-dimensional Cell Tissue Having Vasculature (3)

Using tris as a cationic substance, the polymeric electrolyte, and the extracellular matrix component described in Table 3, respectively, a three-dimensional cell tissue having a vasculature was constructed.

NHDF of 2.0 × 10⁶ cells and HUVEC of 3.0 × 10⁴ cells were suspended in a mixture solution of 150 µL of a solution of 0.2 mg/mL or 0.1 mg/mL of polymeric electrolyte/50 mM tris-hydrochloric acid buffer solution (pH 7.4) and 150 µL of 0.2 mg/mL or 0.1 mg/mL of extracellular matrix component/acetic acid solution (pH 3.7) (that is, each of final concentrations of polymeric electrolyte and extracellular matrix component was 0.1 mg/mL or 0.05 mg/mL). After that, the suspension was centrifuged at room temperature for one minute at 400 × g to obtain a viscous body.

As a comparison in which a polymeric electrolyte and an extracellular matrix component are not used, NHDF of 2.0 × 10⁶ cells and HUVEC of 3.0 × 10⁴ cells were suspended in a mixture solution of 150 µL of 50 mM tris-hydrochloric acid buffer solution (pH 7.4) and 150 µL of acetic acid solution (pH 3.7). After that, the suspension was centrifuged at room temperature for one minute at 400 × g to obtain a cell aggregate.

In addition, as a comparison in which a cationic substance is not used, NHDF of 2.0 × 10⁶ cells and HUVEC of 3.0 × 10⁴ cells were suspended in a mixture solution of 150 µL of DMEM and 150 µL of acetic acid solution (pH 3.7). After that, the suspension was centrifuged at room temperature for one minute at 400 × g to obtain a cell aggregate.

The obtained cell aggregate was suspended in a DMEM containing 10% FBS, and the obtained suspension was sown into a 24 well cell culture insert and centrifuged at room temperature for one minute at 400 × g. Subsequently, a DMEM containing 10% FBS was added to the cell culture insert, and culture was performed in a CO₂ incubator (37°C, 5% CO₂) for five days. After the culture, using an anti-CD31 antibody (monoclonal mouse anti-human CD31 antibody, Clone JC70A, Code M0823, Dako) as a primary antibody, and goat anti-mouse IgG-AlexaFluor (registered trademark) 594 (invitrogen (trademark)) as a secondary antibody, the constructed tissue was subjected to immune stain. The immune stain was performed by a known method. As a result, in all evaluation slices, a vascular network was formed in the tissue.

As a result, in all evaluation slices, that is, in both of the tissue constructed from a cell suspension in which each of the final concentrations of the polymeric electrolyte and the extracellular matrix component was 0.1 mg/mL and the tissue constructed from a cell suspension in which each of the final concentrations of both components was 0.05 mg/mL, regardless of the kind of the polymeric electrolyte and the extracellular matrix component, a vascular network was formed in the tissue. However, although the tissue constructed by not using all the three components and the tissue constructed by using only tris were three-dimensional cell tissues in which a vascular network was formed immediately after construction, adhesion between cells was low, and thus the cells were loosened and the tissue was collapsed during culture medium exchange.

Subsequently, the density of the cell layer of each of the constructed three-dimensional cell tissues was measured.

Specifically, NHDF of 3.5 × 10⁶ cells were suspended in a mixture solution of 250 µL of 0.2 mg/mL or 0.1 mg/mL of polymeric electrolyte/50 mM tris-hydrochloric acid buffer solution (pH 7.4) and 250 µL of 0.2 mg/mL or 0.1 mg/mL of extracellular matrix component/acetic acid solution (pH 3.7) (that is, each of the final concentrations of the polymeric electrolyte and the extracellular matrix component was 0.1 mg/mL or 0.05 mg/mL). After that, the suspension was centrifuged at room temperature for one minute at 400 × g to obtain a viscous body. The obtained viscous body was suspended in a DMEM containing 10% FBS, and the obtained suspension was sown into a 24 well cell culture insert and centrifuged at room temperature for one minute at 400 × g. Subsequently, a DMEM containing 10% FBS was added to the cell culture insert, and culture was performed in a CO₂ incubator (37°C, 5% CO₂) for 24 hours. After the culture, a constructed tissue was fixed with 10% formalin, and immersed with 70% ethanol to prepare a paraffin embedding slice. Preparation of the paraffin embedding slice was performed by a known method. The prepared slice was subjected to HE stain. The HE stain was performed by a known method.

From each HE-stained evaluation slice, a maximum thickness of each of the constructed three-dimensional cell tissues was measured. As a result, the tissue constructed by not mixing three components of a cationic substance, a polymeric electrolyte, and an extracellular matrix component had the thinnest maximum thickness. The maximum thickness of the tissue constructed by containing at least one of a cationic substance, a polymeric electrolyte, and an extracellular matrix component was equal to or greater than that of the tissue constructed by not mixing three components.

In addition, from each HE-stained evaluation slice, a density of a cell layer of each of the constructed three-dimensional cell tissues was measured. Specifically, first, a maximum value of the number of cell layers of the tissue in each evaluation slice and a thickness (µm) of a portion on which the number of cell layers becomes maximum were measured. From the maximum value of the number of the cell layers and the measurement value of the thickness, the number of cell layers per thickness of 10 µm was calculated.

Table 3 shows the result of an evaluation slice of the tissue constructed from the cell suspension in which each of the final concentrations of the polymeric electrolyte and the extracellular matrix component was 0.1 mg/mL. Here, in a case where laminin was used as an extracellular matrix component and polystyrene sulfonic acid was used as a polymeric electrolyte, Table 3 shows the result of the tissue in which each of the final concentrations in a mixture solution was 0.05 mg/mL.

The upper row of Table 3 shows a relative value of the maximum thickness of each tissue in a case where the thickness (80 µm) of the tissue constructed by not mixing all the three components was 1. In the middle row of Table 3, the calculated number of cell layers per thickness of 10 µm is shown. The lower row of Table 3 shows the number of cell layers obtained by subtracting the number of cell layers per thickness of 10 µm of the tissue constructed by not using all the three components from the calculated number of cell layers per thickness of 10 µm. Since only the tissue constructed by mixing cells with tris, heparin, and collagen was treated with three specimens, Table 3 shows the result of all the three specimens. In the table, "Tris" represents tris-hydrochloric acid buffer solution, "PSS" represents polystyrene sulfonic acid, "none" in the column of "extracellular matrix" means that an extracellular matrix was not added, and "none" in the vertical axis means that none of tris and polymeric electrolyte was added.

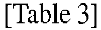

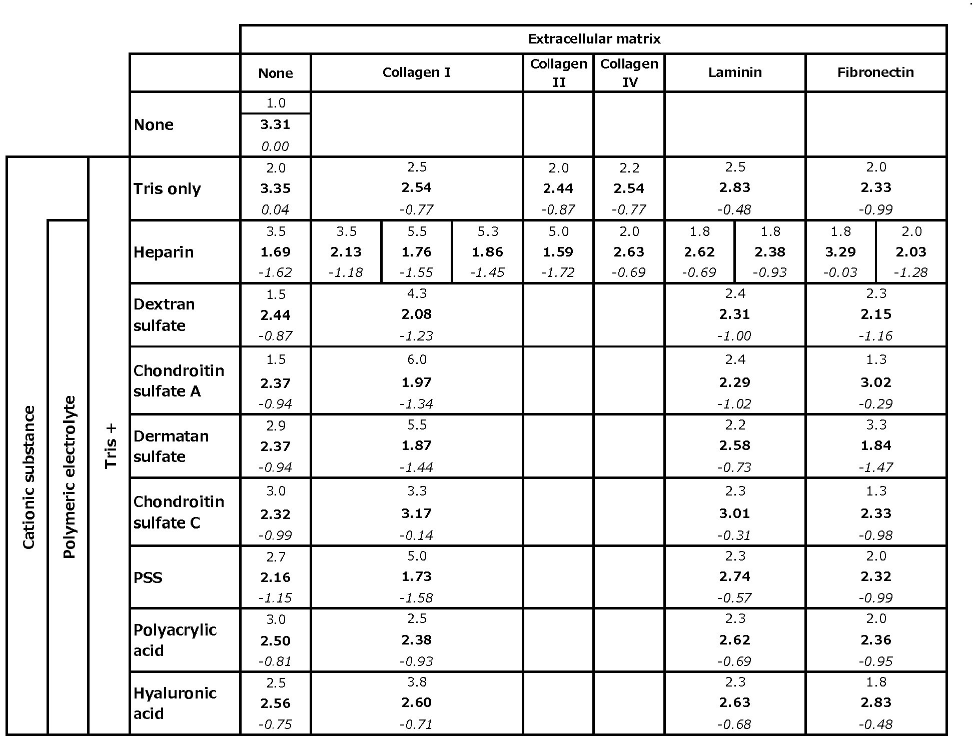

In addition, from each HE-stained evaluation slice, the number of cells of each of the three-dimensional cell tissues constructed by using the method according to the embodiment was counted.

Table 4 shows the result of an evaluation slice of the tissue constructed from the cell suspension in which each of the final concentrations of the polymeric electrolyte and the extracellular matrix component was 0.1 mg/mL. Here, Table 4 shows the result of the tissue in which each of the final concentrations in the mixture solution was obtained as 0.05 mg/mL, in a case where a tris and collagen I was used as an extracellular matrix component without using a polymeric electrolyte, in a case where laminin was used as an extracellular matrix component, and dextran sulfate, chondroitin sulfate A, dermatan sulfate, chondroitin sulfate C, polystyrene sulfonic acid, and polyacrylic acid were used as a polymeric electrolyte, in a case where collagen I was used as an extracellular matrix component, and chondroitin sulfate C was used as a polymeric electrolyte, in a case where collagen IV was used as an extracellular matrix component and heparin was used as a polymeric electrolyte, and in a case where fibronectin was used as an extracellular matrix component and polyacrylic acid was used as a polymeric electrolyte.

The upper row of Table 4 shows a relative value of a thickness of an area where the number of cells of each tissue was counted, based on the thickness of an area where the number of cells of the tissue constructed by not mixing all the three components was set as 1. In the middle row, the calculated number of cells per area of 100 µm in the thickness direction and 50 µm in the width direction is shown. The lower row of Table 4 shows the number of cells obtained by subtracting the number of cells per the above-mentioned area of the tissue constructed by not using all the three components from the number of cells per the area.

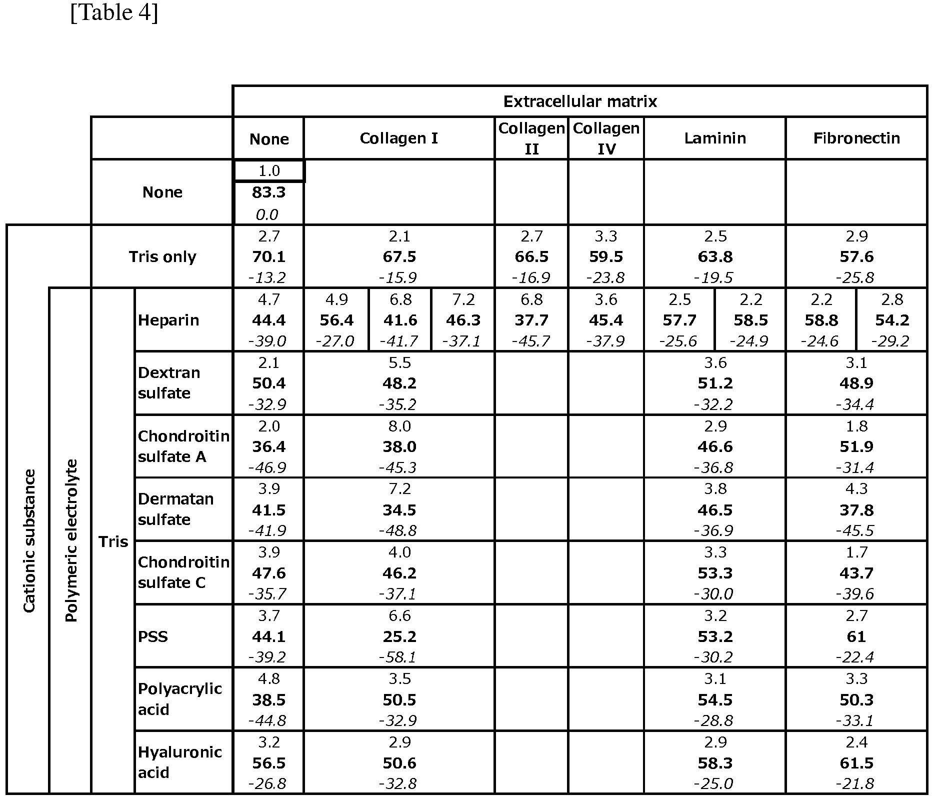

As a result, the tissue constructed by using tris and an extracellular matrix component and the tissue constructed by using tris, a polymeric electrolyte, and an extracellular matrix component had almost no voids inside and were significantly favorable three-dimensional cell tissues, in spite of having a sufficient thickness. On the contrary, in the tissue constructed by not using an extracellular matrix component, there existed a lot of voids inside the tissue. In addition, in the tissue constructed by not using all the three components and the tissue constructed by using only tris, adhesion between cells was low, and thus the cells were loosened and the tissue was collapsed during culture medium exchange. On the contrary, regarding the tissue containing at least any of a polymeric electrolyte and an extracellular matrix component, it was possible to perform culture medium exchange without a problem and to perform culture for a long period of time.

The tissue constructed by using only tris had the maximum thickness about two times that of the tissue constructed by not using all the three components. However, the tissue constructed by using only tris had the same level of the densities of the cell layers and the cells as those of the tissue constructed by not using all the three components. On the contrary, the tissue containing at least any of a polymeric electrolyte and an extracellular matrix component along with tris had the maximum thickness 1.3 to 5.5 times that of the tissue constructed by not using all the three components, but had a lower densities of the cell layers and the cells than those of the tissue constructed by not using all the three components. In an evaluation slice of the tissue constructed from the cell suspension in which each of the final concentrations of the polymeric electrolyte and the extracellular matrix component was 0.05 mg/mL, almost the same result was obtained. From the result, it was recognized that, by mixing cells with at least an extracellular matrix component in addition to a cationic substance, a three-dimensional cell tissue with a low density of the cell layers and a certain thickness was obtained.

When the values of Table 3 and Table 4 are compared with each other, it was recognized that the tissue not containing a polymeric electrolyte and containing a tris and an extracellular matrix component had smaller number of layers per 10 µm and almost the same number of cells than the tissue constructed by using only tris. It is considered that this is because there are a lot of cells in which the height of the cell nucleus is at the same position (position seen to be the same layer), in the tissue. In other words, it is considered that this is because, in the tissue, a lot of cells at the position at which the cell (nucleus) is seen to be the same layer are in a width direction (horizontal direction) of the tissue. For this reason, in the tissue not containing a polymeric electrolyte and containing a tris and an extracellular matrix component, cells easily gather on the same layer compared to the tissue constructed by using only tris, whereas in the tissue constructed by using only tris, cells closely gather in the gravity direction.

Although the cells in vivo are surrounded by matrix components, the cells in vivo exist by being adjacent to one another without being randomly scattered. In view of this point, according to the present invention, it is possible to construct a three-dimensional tissue in which cells are in a state of being appropriately adjacent to one another and are not collapsed in the gravity direction.

According to the present invention, it is possible to produce a thicker three-dimensional cell tissue in a faster and convenient manner. Therefore, the present invention is useful in the field of regeneration medicine. In addition, the present invention is useful in the development of pharmaceutical products.

## Claims

1. A method of producing a three-dimensional cell tissue, comprising:
a step A of mixing cells with a cationic substance, an extracellular matrix component, and a polymeric electrolyte to obtain a mixture;
a step B of gathering the cells from the obtained mixture to form a cell aggregate on a substrate; and
a step C of culturing the cells to obtain a three-dimensional cell tissue,
wherein the polymeric electrolyte is selected from the group consisting of glycosaminoglycan, dextran sulfate, rhamnan sulfate, fucoidan, carrageenan, polystyrene sulfonic acid, polyacrylamide-2-methylpropanesulfonic acid, polyacrylic acid, and a combination thereof,
the extracellular matrix component is selected from the group consisting of collagen, laminin, fibronectin, vitronectin, elastin, tenascin, entactin, fibrillin, proteoglycan, and a combination thereof,
the cationic substance is a tris-hydrochloric acid buffer solution, a tris-maleic acid buffer solution, a bis-tris-buffer solution, or HEPES,
a concentration of the polymeric electrolyte is more than 0 mg/mL and less than 1.0 mg/mL, preferably from 0.05 mg/mL to 0.1 mg/mL, and
a concentration of the extracellular matrix component is more than 0 mg/mL and less than 1.0 mg/mL, and preferably from 0.05 mg/mL to 0.1 mg/mL.

2. The method of producing a three-dimensional cell tissue according to Claim 1, further comprising:
a step A'-1 of removing a liquid portion from the obtained mixture to obtain a cell aggregate, and a step A'-2 of suspending the cell aggregate in a solution to obtain a suspension, after the step A; and
a step B' of precipitating the cells from the obtained suspension to form a cell precipitate on the substrate, instead of the step B.

3. The method of producing a three-dimensional cell tissue according to Claim 1 or 2, wherein
in the step B or the step B', a method for gathering the cells is centrifugal separation, magnetic separation, or filtration.

4. The method of producing a three-dimensional cell tissue according to any one of Claims 1 to 3, wherein
the cell aggregate in the step B or the cell precipitate in the step B' is in a layer shape.

5. The method of producing a three-dimensional cell tissue according to any one of Claims 1 to 4, wherein
a mixture ratio of the polymeric electrolyte to the extracellular matrix component is 1:2 to 2:1.

6. The method of producing a three-dimensional cell tissue according to any one of Claims 1 to 5, wherein
the cells in the step A are a plurality of kinds of cells.

7. The method of producing a three-dimensional cell tissue according to Claim 6, wherein
the plurality of kinds of cells are selected from the group consisting of nerve cells, dendritic cells, immune cells, vascular endothelial cells, lymphatic endothelial cells, fibroblasts, cancer cells, cancer stem cells, epithelial cells, myocardial cells, liver cells, pancreatic islet cells, tissue stem cells, iPS cells, ES cells, and smooth muscle cells.

8. The method of producing a three-dimensional cell tissue according to any one of Claims 1 to 7, wherein
a thickness of the obtained three-dimensional cell tissue is 5 to 500 µm.

9. The method of producing a three-dimensional cell tissue according to any one of Claims 1 to 8, wherein
the number of cell layers in the obtained three-dimensional cell tissue is 1 to 100 layers, and preferably from 10 to about 100 layers.

10. The method of producing a three-dimensional cell tissue according to any one of Claims 1 to 9, wherein
the number of cells per area of 100 µm in a thickness direction and of 50 µm in a width direction in a region including a position in which a thickness of the obtained three-dimensional cell tissue is the maximum is 70 or less.

11. The method of producing a three-dimensional cell tissue according to any one of Claims 1 to 10, wherein
the obtained three-dimensional cell tissue includes a plurality of kinds of cells.

12. The method of producing a three-dimensional cell tissue according to any one of Claims 1 to 11, wherein
the obtained three-dimensional cell tissue has a vasculature.

13. Use of a kit for performing the method according to any one of Claims 1 to 12, the kit comprises:
the cationic substance, the polymeric electrolyte and the extracellular matrix component.

## Patentansprüche

1. Verfahren zur Herstellung eines dreidimensionalen Zellgewebes, umfassend:
einen Schritt A des Mischens von Zellen mit einer kationischen Substanz, einer extrazellulären Matrixkomponente und einem polymeren Elektrolyten, um eine Mischung zu erhalten;
einen Schritt B des Sammelns der Zellen aus der erhaltenen Mischung, um ein Zellaggregat auf einem Substrat zu bilden; und
einen Schritt C der Kultivierung der Zellen, um ein dreidimensionales Zellgewebe zu erhalten,
wobei der polymere Elektrolyt ausgewählt ist aus der Gruppe bestehend aus Glycosaminoglycan, Dextransulfat, Rhamnansulfat, Fucoidan, Carrageenan, Polystyrolsulfonsäure, Polyacrylamid-2-methylpropansulfonsäure, Polyacrylsäure und einer Kombination davon,
die extrazelluläre Matrixkomponente ausgewählt ist aus der Gruppe bestehend aus Kollagen, Laminin, Fibronektin, Vitronektin, Elastin, Tenascin, Entactin, Fibrillin, Proteoglycan und einer Kombination davon,
die kationische Substanz eine Tris-Salzsäure-Pufferlösung, eine Tris-Maleinsäure-Pufferlösung, eine Bis-Tris-Pufferlösung oder HEPES ist,
eine Konzentration des polymeren Elektrolyten mehr als 0 mg/ml und weniger als 1,0 mg/ml, bevorzugt 0,05 mg/ml bis 0,1 mg/ml, beträgt und
eine Konzentration der extrazellulären Matrixkomponente mehr als 0 mg/ml und weniger als 1,0 mg/ml, bevorzugt von 0,05 mg/ml bis 0,1 mg/ml, beträgt.

2. Verfahren zur Herstellung eines dreidimensionalen Zellgewebes nach Anspruch 1, ferner umfassend:
einen Schritt A'-1 des Entfernens eines flüssigen Anteils aus der erhaltenen Mischung, um ein Zellaggregat zu erhalten, und einen Schritt A'-2 des Suspendierens des Zellaggregats in einer Lösung, um eine Suspension zu erhalten, nach dem Schritt A; und
einen Schritt B' des Ausfällens der Zellen aus der erhaltenen Suspension, um einen Zellpräzipitat auf dem Substrat zu bilden, anstelle des Schritts B.

3. Verfahren zur Herstellung eines dreidimensionalen Zellgewebes nach Anspruch 1 oder 2, wobei
im Schritt B oder im Schritt B' ein Verfahren zum Sammeln der Zellen Zentrifugalseparation, Magnetseparation oder Filtration ist.

4. Verfahren zur Herstellung eines dreidimensionalen Zellgewebes nach einem der Ansprüche 1 bis 3, **dadurch gekennzeichnet, dass**
das Zellaggregat in Schritt B oder das Zellpräzipitat in Schritt B' in einer Schichtform vorliegt.

5. Verfahren zur Herstellung eines dreidimensionalen Zellgewebes nach einem der Ansprüche 1 bis 4, **dadurch gekennzeichnet, dass**
ein Mischungsverhältnis des polymeren Elektrolyten zu der extrazellulären Matrixkomponente 1:2 bis 2:1 beträgt.

6. Verfahren zur Herstellung eines dreidimensionalen Zellgewebes nach einem der Ansprüche 1 bis 5, wobei
die Zellen in Schritt A mehrere Arten von Zellen sind.

7. Verfahren zur Herstellung eines dreidimensionalen Zellgewebes nach Anspruch 6, **dadurch gekennzeichnet, dass**
die mehreren Arten von Zellen ausgewählt sind aus der Gruppe bestehend aus Nervenzellen, dendritischen Zellen, Immunzellen, vaskulären Endothelzellen, lymphatischen Endothelzellen, Fibroblasten, Krebszellen, Krebsstammzellen, Epithelzellen, Herzmuskelzellen, Leberzellen, Pankreasinselzellen, Gewebestammzellen, iPS-Zellen, ES-Zellen und glatten Muskelzellen.

8. Verfahren zur Herstellung eines dreidimensionalen Zellgewebes nach einem der Ansprüche 1 bis 7, wobei
eine Dicke des erhaltenen dreidimensionalen Zellgewebes 5 bis 500 µm beträgt.

9. Verfahren zur Herstellung eines dreidimensionalen Zellgewebes nach einem der Ansprüche 1 bis 8, wobei
die Anzahl der Zellschichten in dem erhaltenen dreidimensionalen Zellgewebe 1 bis 100 Schichten und bevorzugt 10 bis etwa 100 Schichten beträgt.

10. Verfahren zur Herstellung eines dreidimensionalen Zellgewebes nach einem der Ansprüche 1 bis 9, wobei
die Anzahl der Zellen pro Fläche von 100 µm in einer Dickenrichtung und von 50 µm in einer Breitenrichtung in einem Bereich, der eine Position einschließt, in der eine Dicke des erhaltenen dreidimensionalen Zellgewebes das Maximum ist, 70 oder weniger beträgt.

11. Verfahren zur Herstellung eines dreidimensionalen Zellgewebes nach einem der Ansprüche 1 bis 10, wobei
das erhaltene dreidimensionale Zellgewebe mehrere Arten von Zellen enthält.

12. Verfahren zur Herstellung eines dreidimensionalen Zellgewebes nach einem der Ansprüche 1 bis 11, wobei
das erhaltene dreidimensionale Zellgewebe eine Vaskulatur aufweist.

13. Verwendung eines Kits zur Durchführung des Verfahrens nach einem der Ansprüche 1 bis 12, wobei das Kit umfasst:
die kationische Substanz, den polymeren Elektrolyten und die extrazelluläre Matrixkomponente.

## Revendications

1. Procédé de production d'un tissu cellulaire tridimensionnel, comprenant :
une étape A de mélange de cellules avec une substance cationique, un composant de la matrice extracellulaire et un électrolyte polymère pour obtenir un mélange ;
une étape B de collecte des cellules du mélange obtenu pour former un agrégat cellulaire sur un substrat ; et
une étape C de culture des cellules pour obtenir un tissu cellulaire tridimensionnel,
dans lequel l'électrolyte polymère est choisi dans le groupe constitué par le glycosaminoglycane, le sulfate de dextrane, le sulfate de rhamnane, le fucoïdane, le carraghénane, l'acide polystyrène sulfonique, l'acide polyacrylamide-2-méthylpropane sulfonique, l'acide polyacrylique, et une combinaison de ceux-ci,
le composant de la matrice extracellulaire est choisi dans le groupe constitué par le collagène, la laminine, la fibronectine, la vitronectine, l'élastine, la ténascine, l'entactine, la fibrilline, le protéoglycane, et une combinaison de ceux-ci,
la substance cationique est une solution tampon d'acide tris-hydrochlorique, une solution tampon d'acide tris-maléique, une solution tampon bis-tris, ou HEPES,
une concentration de l'électrolyte polymère est supérieure à 0 mg/mL et inférieure à 1,0 mg/mL, de préférence de 0,05 mg/mL à 0,1 mg/mL, et
une concentration du composant de la matrice extracellulaire est supérieure à 0 mg/mL et inférieure à 1,0 mg/mL, et de préférence de 0,05 mg/mL à 0,1 mg/mL.

2. Procédé de production d'un tissu cellulaire tridimensionnel selon la revendication 1, comprenant en outre :
une étape A'-1 d'élimination d'une partie liquide du mélange obtenu pour obtenir un agrégat cellulaire, et une étape A'-2 de mise en suspension de l'agrégat cellulaire dans une solution pour obtenir une suspension, après l'étape A ; et
une étape B' de précipitation des cellules de la suspension obtenue pour former un précipité cellulaire sur le substrat, au lieu de l'étape B.

3. Procédé de production d'un tissu cellulaire tridimensionnel selon la revendication 1 ou 2, dans lequel
dans l'étape B ou dans l'étape B', un procédé de collecte des cellules est une séparation centrifuge, une séparation magnétique ou une filtration.

4. Procédé de production d'un tissu cellulaire tridimensionnel selon l'une quelconque des revendications 1 à 3, dans lequel
l'agrégat cellulaire de l'étape B ou le précipité cellulaire de l'étape B' prend la forme d'une couche.

5. Procédé de production d'un tissu cellulaire tridimensionnel selon l'une quelconque des revendications 1 à 4, dans lequel
un rapport de mélange de l'électrolyte polymère au composant de la matrice extracellulaire est de 1:2 à 2:1.

6. Procédé de production d'un tissu cellulaire tridimensionnel selon l'une quelconque des revendications 1 à 5, dans lequel
les cellules de l'étape A sont une pluralité de types de cellules.

7. Procédé de production d'un tissu cellulaire tridimensionnel selon la revendication 6, dans lequel
la pluralité de types de cellules est choisie dans le groupe constitué par les cellules nerveuses, les cellules dendritiques, les cellules immunitaires, les cellules endothéliales vasculaires, les cellules endothéliales lymphatiques, les fibroblastes, les cellules cancéreuses, les cellules souches cancéreuses, les cellules épithéliales, les cellules myocardiques, les cellules hépatiques, les cellules d'îlots pancréatiques, les cellules souches tissulaires, les cellules iPS, les cellules ES et les cellules musculaires lisses.

8. Procédé de production d'un tissu cellulaire tridimensionnel selon l'une quelconque des revendications 1 à 7, dans lequel
une épaisseur du tissu cellulaire tridimensionnel obtenu est de 5 à 500 µm.

9. Procédé de production d'un tissu cellulaire tridimensionnel selon l'une quelconque des revendications 1 à 8, dans lequel
le nombre de couches cellulaires dans le tissu cellulaire tridimensionnel obtenu est de 1 à 100 couches, et de préférence de 10 à environ 100 couches.

10. Procédé de production d'un tissu cellulaire tridimensionnel selon l'une quelconque des revendications 1 à 9, dans lequel
le nombre de cellules par surface de 100 µm dans une direction d'épaisseur et de 50 µm dans une direction de largeur dans une zone comprenant une position dans laquelle l'épaisseur du tissu cellulaire tridimensionnel obtenu est maximale est 70 ou moins.

11. Procédé de production d'un tissu cellulaire tridimensionnel selon l'une quelconque des revendications 1 à 10, dans lequel
le tissu cellulaire tridimensionnel obtenu comprend une pluralité de types de cellules.

12. Procédé de production d'un tissu cellulaire tridimensionnel selon l'une quelconque des revendications 1 à 11, dans lequel
le tissu cellulaire tridimensionnel obtenu a une vascularisation.

13. Utilisation d'un kit pour réaliser le procédé selon l'une quelconque des revendications 1 à 12, le kit comprenant :
la substance cationique, l'électrolyte polymère et le composant de la matrice extracellulaire.
